# EUROPEAN PATENT SPECIFICATION

(11) **EP 4 213 708 B1**
(45) Date of publication and mention of the grant of the patent: **10.06.2026**
(21) Application number: 21790986.0
(22) Date of filing: 17.09.2021
(51) Int. Cl.: A61B 5/00, A61B 5/01, A61B 5/021, A61B 5/024, A61B 5/0531, A61B 5/08, A61B 5/087, A61B 5/091, A61B 5/107, A61B 5/11, A61B 5/1455, A61B 5/318, A61B 5/361, A61B 5/363, A61B 5/369, A61M 16/00

(54) **SYSTEM FOR MONITORING COMORBIDITIES**
SYSTEM ZUR ÜBERWACHUNG VON KOMORBIDITÄTEN
SYSTÈME DE SURVEILLANCE DE COMORBIDITÉS

(30) Priority: 18.09.2020 US 202063080344 P
(43) Date of publication of application: 26.07.2023
(73) Proprietor: ResMed Sensor Technologies Limited, Sandyford D18 T6T7 Dublin (IE)
(72) Inventor: HOLLEY, Liam, Bella Vista, New South Wales 2153 (AU); VITHANAGE FERNANDO, Varuni Lakshana, Bella Vista, New South Wales 2153 (AU); CERINA, Luca, Dublin, D18 T6T7 (IE); CHAH, Ehsan, Dublin, D18 T6T7 (IE)
(74) Representative: Mathys & Squire
(86) International application number: PCT/IB2021/058513
(87) International publication number: WO 2022/058964

(56) References cited:
- WO-A2-2020/092701
- US-A1- 2009 203 972
- US-A1- 2016 135 734

## Description

### CROSS-REFERENCE TO RELATED APPLICATIONS

This application claims the benefit of, and priority to, U.S. Provisional Patent Application No. 63/080,344 filed on September 18, 2020.

### TECHNICAL FIELD

The present disclosure relates generally to systems for analyzing data generated during an individual's use of a respiratory therapy system, and more particularly, to systems for analyzing data generated during the user's use of a respiratory therapy system to determine a first metric associated with sleep-disordered breathing and a second metric associated with a health condition other sleep-disordered breathing.

### BACKGROUND

Many individuals suffer from sleep-related and/or respiratory disorders such as, for example, Sleep-Disordered Breathing (SDB), which can include Obstructive Sleep Apnea (OSA), Central Sleep Apnea (CSA), other types of apneas such as mixed apneas and hypopneas, and Respiratory Effort Related Arousal (RERA). These individuals may also suffer from other health conditions (which may be referred to as comorbidities), such as insomnia (characterized by, for example, difficult in initiating sleep, frequent or prolonged awakenings after initially falling asleep, and/or an early awakening with an inability to return to sleep), Periodic Limb Movement Disorder (PLMD), Restless Leg Syndrome (RLS), Cheyne-Stokes Respiration (CSR), respiratory insufficiency, Obesity Hyperventilation Syndrome (OHS), Chronic Obstructive Pulmonary Disease (COPD), Neuromuscular Disease (NMD), rapid eye movement (REM) behavior disorder (also referred to as RBD), dream enactment behavior (DEB), hypertension, diabetes, stroke, , and chest wall disorders. It can be difficult to accurately identify and monitor these other health conditions. In individuals using a respiratory therapy system to treat the sleep-related disorder, the use of the respiratory therapy system can impact the severity of these health conditions. Thus, it would be advantageous to be able to analyze data associated with the individual's use of the respiratory therapy system to determine the identity, severity, and/or progression of any other health conditions. The present disclosure is directed to systems, devices, and methods to allow for identification and/or monitoring of health conditions using data associated with the individual's use of a respiratory therapy system

A system according to the prior art is known from WO 2020/092701.

### SUMMARY

According to some implementations of the present disclosure, a system for monitoring a sleep session of a user comprises a respiratory therapy system, a memory device, and a control system. The respiratory therapy system includes a respiratory therapy device and a user interface. The respiratory therapy device is configured to supply pressurized air. The respiratory therapy device includes a housing having one or more sensors positioned therein. The user interface is coupled to the respiratory therapy device via a conduit, and is configured to engage the user and aid in directing the supplied pressurized air to the airway of the user. The memory device stores machine-readable instructions. The control system is coupled to the memory device, and includes one or more processors configured to execute the machine-readable instructions to: cause data associated with the user to be generated, during a current sleep session, the data including at least a first portion that is generated by the one or more sensors that are positioned in the housing of the respiratory therapy device; analyze the generated data to determine a value of a first metric that is associated with a sleep disordered breathing (SDB) condition; analyze the generated data to determine a value of a second metric that is associated with a health condition other than the SDB condition, the health condition including insomnia, wherein the second metric includes a total time in bed, a total sleep time, a total wake time, a sleep onset latency, a wake-after-sleep-onset parameter, a sleep efficiency, a fragmentation index, an amount of time to fall asleep, a consistency of breathing rate, a fall asleep time, a wake time, a rate of sleep disturbances, a number of movements, or any combination thereof; in response to the determined value of the second metric indicating a presence of insomnia, cause an action to be performed, the action including adjusting one or more settings of the respiratory therapy system. The adjusting includes: (i) decreasing a pressure of the pressurized air below a desired therapy pressure, and in response to determining that the user has fallen asleep, increasing the pressure of the pressurized air to at least the desired therapy pressure; and/or (ii) increasing a ramp time of the pressurized air, where the ramp time is the time it takes for the pressure of the air to increase to the desired therapy pressure from the beginning of the use of the respiratory therapy system.

According to some implementations, the one or more processors of the control system are further configured to execute the machine-readable instructions to: track the second metric over a plurality of sleep sessions; determine the value of the second metric for each of the plurality of sleep sessions; determine (i) a duration of use of the respiratory therapy system for each of the plurality of sleep sessions, (ii) values of one or more settings of the respiratory therapy system for each of the plurality of sleep session, (iii) or both; and identify (i) an optimal duration of use of the respiratory therapy system, (ii) optimal values of the one or more settings of the respiratory therapy system, or (iii) both, to treat the health condition.

According to some implementations, the one or more processors of the control system are further configured to execute the machine-readable instructions to: receive historical data associated with one or more prior sleep sessions of the user; analyze the historical data to determine a value of the second metric for each of the one or more prior sleep sessions; and compare the determined value of the second metric for each of the one or more prior sleep sessions to the determined value of the second metric for the current sleep session.

According to some implementations, the one or more processors of the control system are further configured to execute the machine-readable instructions to: determine a severity of the health condition based at least in part on the determined value of the second metric for the current sleep session; generate data during one or more subsequent sleep sessions; determine a value of the second metric for each of the one or more subsequent sleep sessions; determine an updated severity of the health condition based at least in part on the determined values of the second metric for each of the one or more subsequent sleep sessions; and, based at least in part on the updated severity of the health condition, cause the action to be performed

According to some implementations, the one or more processors of the control system are further configured to execute the machine-readable instructions to: receive additional data associated with the user while awake; analyze the additional data to determine a value of a third metric associated with the health condition; and cause the action to be performed based at least in part on the determined value of the third metric associated with the health condition

Also disclosed herein is a method of monitoring a sleep session of a user comprises generating data, during a current sleep session, associated with an individual of a respiratory therapy system. The method further includes analyzing the generated data to determine a value of a first metric that is associated with a sleep disordered breathing (SDB) condition. The method further includes analyzing the generated data to determine a value of a second metric that is associated with a health condition other than the SDB condition. The method further includes, based at least in part on the determined value of the second metric, causing an action to be performed.

The method also disclosed herein further includes tracking the second metric over a plurality of sleep sessions. The method further includes determining the value of the second metric for each of the plurality of sleep sessions. The method further includes determining (i) a duration of use of the respiratory therapy system for each of the plurality of sleep sessions, (ii) values of one or more settings of the respiratory therapy system for each of the plurality of sleep session, (iii) or both. The method further includes identifying (i) an optimal duration of use of the respiratory therapy system, (ii) optimal values of the one or more settings of the respiratory therapy system, or (iii) both, to treat the health condition.

The method also disclosed herein further includes receiving historical data associated with one or more prior sleep sessions of the user. The method further includes analyzing the historical data to determine a value of the second metric for each of the one or more prior sleep sessions. The method further includes comparing the determined value of the second metric for each of the one or more prior sleep sessions to the determined value of the second metric for the current sleep session.

The method also disclosed herein further includes determining a severity of the health condition based at least in part on the determined value of the second metric for the current sleep session. The method further includes generating data during one or more subsequent sleep sessions. The method further includes determining a value of the second metric for each of the one or more subsequent sleep sessions. The method further includes determining an updated severity of the health condition based at least in part on the determined values of the second metric for each of the one or more subsequent sleep sessions. The method further includes, based at least in part on the updated severity of the health condition, causing an action to be performed.

The method also disclosed herein further includes receiving additional data associated with the user while awake. The method further includes analyzing the additional data to determine a value of a third metric associated with the health condition. The method further includes causing the action to be performed based at least in part on the determined value of the third metric associated with the health condition.

The above summary is not intended to represent each implementation or every aspect of the present disclosure. Additional features and benefits of the present disclosure are apparent from the detailed description and figures set forth below.

A system according to the invention is defined in claim 1.

### BRIEF DESCRIPTION OF THE DRAWINGS

FIG. 1 is a functional block diagram of a system for monitoring a sleep session, according to some implementations of the present disclosure;
FIG. 2 is a perspective view of the system of FIG. 1, a user of the system, and a bed partner of the user, according to some implementations of the present disclosure;
FIG. 3 illustrates an exemplary timeline for a sleep session, according to some implementations of the present disclosure;
FIG. 4 illustrates an exemplary hypnogram associated with the sleep session of FIG. 3, according to some implementations of the present disclosure;
FIG. 5 is a process flow diagram for a first method of monitoring an individual during a sleep session as the user uses a respiratory therapy system, according to some implementations of the present disclosure;
FIG. 6 is a process flow diagram for a second method of monitoring an individual during a sleep session as the user uses a respiratory therapy system, according to some implementations of the present disclosure; and
FIG. 7 is a process flow diagram for a third method of monitoring an individual during a sleep session as the user uses a respiratory therapy system, according to some implementations of the present disclosure.

### DETAILED DESCRIPTION

The present disclosure is described with reference to the attached figures, where like reference numerals are used throughout the figures to designate similar or equivalent elements. The figures are not drawn to scale, and are provided merely to illustrate the instant disclosure. Several aspects of the disclosure are described below with reference to example applications for illustration.

Many individuals suffer from sleep-related and/or respiratory disorders, such as Periodic Limb Movement Disorder (PLMD), Restless Leg Syndrome (RLS), Sleep-Disordered Breathing (SDB) such as Obstructive Sleep Apnea (OSA), Central Sleep Apnea (CSA) and other types of apneas, Respiratory Effort Related Arousal (RERA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), and chest wall disorders. Obstructive Sleep Apnea (OSA), a form of Sleep Disordered Breathing (SDB), is characterized by events including occlusion or obstruction of the upper air passage during sleep resulting from a combination of an abnormally small upper airway and the normal loss of muscle tone in the region of the tongue, soft palate and posterior oropharyngeal wall. Central Sleep Apnea (CSA) is another form of sleep disordered breathing. CSA results when the brain temporarily stops sending signals to the muscles that control breathing. Other types of apneas include hypopnea, hyperpnea, and hypercapnia. Hypopnea is generally characterized by slow or shallow breathing caused by a narrowed airway, as opposed to a blocked airway. Hyperpnea is generally characterized by an increase depth and/or rate of breathing. Hypercapnia is generally characterized by elevated or excessive carbon dioxide in the bloodstream, typically caused by inadequate respiration. A Respiratory Effort Related Arousal (RERA) event is typically characterized by an increased respiratory effort for ten seconds or longer leading to arousal from sleep and which does not fulfill the criteria for an apnea or hypopnea event. RERAs are defined as a sequence of breaths characterized by increasing respiratory effort leading to an arousal from sleep, but which does not meet criteria for an apnea or hypopnea. These events must fulfil both of the following criteria: (1) a pattern of progressively more negative esophageal pressure, terminated by a sudden change in pressure to a less negative level and an arousal, and (2) the event lasts ten seconds or longer. In some implementations, a Nasal Cannula/Pressure Transducer System is adequate and reliable in the detection of RERAs. A RERA detector may be based on a real flow signal derived from a respiratory therapy device. For example, a flow limitation measure may be determined based on a flow signal. A measure of arousal may then be derived as a function of the flow limitation measure and a measure of sudden increase in ventilation. One such method is described in WO 2008/138040 and U.S. Patent No. 9,358,353, assigned to ResMed Ltd..

Cheyne-Stokes Respiration (CSR) is a further form of SDB. CSR is a disorder of a patient's respiratory controller in which there are rhythmic alternating periods of waxing and waning ventilation known as CSR cycles. CSR is characterized by repetitive de-oxygenation and re-oxygenation of the arterial blood. OHS is defined as the combination of severe obesity and awake chronic hypercapnia, in the absence of other known causes for hypoventilation. Symptoms include dyspnea, morning headache and excessive daytime sleepiness. COPD encompasses any of a group of lower airway diseases that have certain characteristics in common, such as increased resistance to air movement, extended expiratory phase of respiration, and loss of the normal elasticity of the lung. NMD encompasses many diseases and ailments that impair the functioning of the muscles either directly via intrinsic muscle pathology, or indirectly via nerve pathology. Chest wall disorders are a group of thoracic deformities that result in inefficient coupling between the respiratory muscles and the thoracic cage.

Many of these disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that can occur when the individual is sleeping. A wide variety of types of data can be used to monitor the health of individuals having any of the above types of sleep-related and/or respiratory disorders (or other disorders). However, it is often difficult to collect accurate data in a manner that does not interrupt or disturb the user's sleep, or interfere with any treatment the user may be undergoing during sleep. Thus, it is advantageous to utilize a system for treatment that includes various sensors to generate and collect data, without disturbing the user, the user's sleep, or the user's treatment.

The Apnea-Hypopnea Index (AHI) is an index used to indicate the severity of sleep apnea during a sleep session. The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild sleep apnea. An AHI that is greater than or equal to 15, but less than 30 is considered indicative of moderate sleep apnea. An AHI that is greater than or equal to 30 is considered indicative of severe sleep apnea. In children, an AHI that is greater than 1 is considered abnormal. Sleep apnea can be considered "controlled" when the AHI is normal, or when the AHI is normal or mild. The AHI can also be used in combination with oxygen desaturation levels to indicate the severity of Obstructive Sleep Apnea.

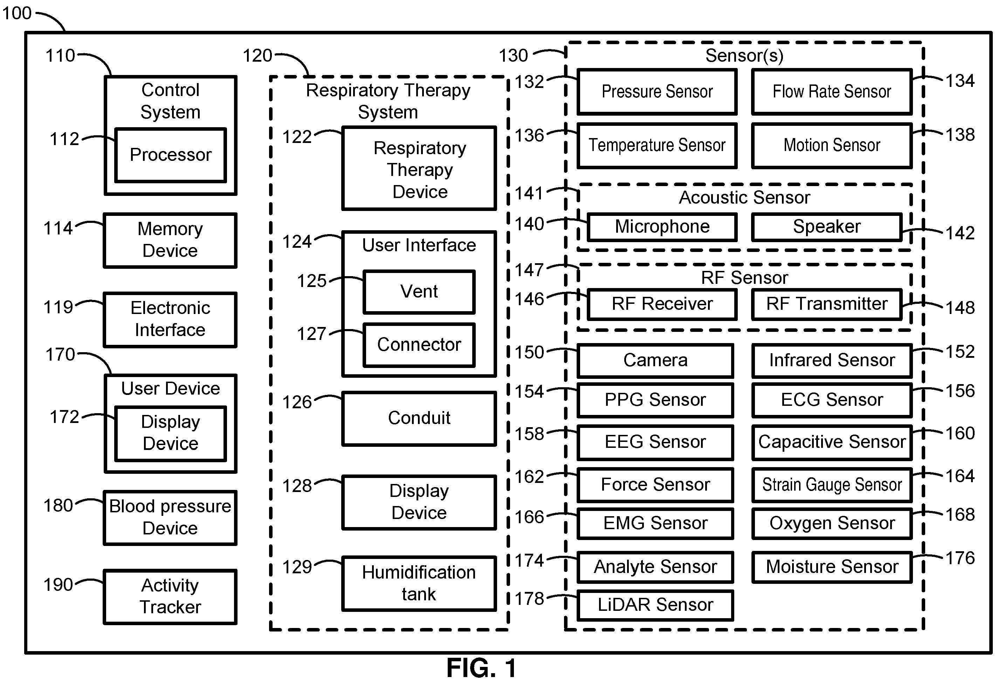

Referring to FIG. 1, a system 100, according to some implementations of the present disclosure, is illustrated. The system 100 can be used to detect, identify, treat, and/or monitor respiratory conditions (and/or other conditions), such as before the manifestation of any external physical symptoms of those conditions. The system 100 includes a control system 110, a memory device 114, an electronic interface 119, one or more sensors 130, and optionally one or more user devices 170. In some implementation, the system 100 further includes a respiratory therapy system 120 that includes a respiratory therapy device 122.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 (or any other control system) or a portion of the control system 110 such as the processor 112 (or any other processor(s) or portion(s) of any other control system), can be used to carry out one or more steps of any of the methods described and/or claimed herein. The control system 110 can be coupled to and/or positioned within, for example, a housing of the user device 170, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the respiratory therapy device 122 of the respiratory therapy system 120, within a housing of the user device 170, within a housing of one or more of the sensors 130, or any combination thereof. Like the control system 110, the memory device 114 can be centralized (within one such housing) or decentralized (within two or more of such housings, which are physically distinct).

In some implementations, the memory device 114 stores a user profile associated with the user. The user profile can include, for example, demographic information associated with the user, biometric information associated with the user, medical information associated with the user, self-reported user feedback, sleep parameters associated with the user (e.g., sleep-related parameters recorded from one or more earlier sleep sessions), or any combination thereof. The demographic information can include, for example, information indicative of an age of the user, a gender of the user, a race of the user, a family medical history (such as a family history of insomnia or sleep apnea), an employment status of the user, an educational status of the user, a socioeconomic status of the user, or any combination thereof. The medical information can include, for example, information indicative of one or more medical conditions associated with the user, medication usage by the user, or both. The medical information data can further include a fall risk assessment associated with the user (e.g., a fall risk score using the Morse fall scale), a multiple sleep latency test (MSLT) result or score and/or a Pittsburgh Sleep Quality Index (PSQI) score or value. The self-reported user feedback can include information indicative of a self-reported subjective sleep score (e.g., poor, average, excellent), a self-reported subjective stress level of the user, a self-reported subjective fatigue level of the user, a self-reported subjective health status of the user, a recent life event experienced by the user, or any combination thereof.

The electronic interface 119 is configured to receive data (e.g., physiological data and/or acoustic data) from the one or more sensors 130 such that the data can be stored in the memory device 114 and/or analyzed by the processor 112 of the control system 110. The electronic interface 119 can communicate with the one or more sensors 130 using a wired connection or a wireless connection (e.g., using an RF communication protocol, a WiFi communication protocol, a Bluetooth communication protocol, an IR communication protocol, over a cellular network, over any other optical communication protocol, etc.). The electronic interface 119 can include an antenna, a receiver (e.g., an RF receiver), a transmitter (e.g., an RF transmitter), a transceiver, or any combination thereof. The electronic interface 119 can also include one more processors and/or one more memory devices that are the same as, or similar to, the processor 112 and the memory device 114 described herein. In some implementations, the electronic interface 119 is coupled to or integrated in the user device 170. In other implementations, the electronic interface 119 is coupled to or integrated (e.g., in a housing) with the control system 110 and/or the memory device 114.

As noted above, in some implementations, the system 100 optionally includes a respiratory therapy system 120 (also referred to as a respiratory pressure therapy system). The respiratory therapy system 120 can include a respiratory therapy device 122 (also referred to as a respiratory pressure device), a user interface 124 (also referred to as a mask or a patient interface), a conduit 126 (also referred to as a tube or an air circuit), a display device 128, a humidification tank 129, or any combination thereof. In some implementations, the control system 110, the memory device 114, the display device 128, one or more of the sensors 130, and the humidification tank 129 are part of the respiratory therapy device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory therapy system 120 is generally used to treat individuals suffering from one or more sleep-related respiratory disorders (e.g., obstructive sleep apnea, central sleep apnea, or mixed sleep apnea), other respiratory disorders such as COPD, or other disorders leading to respiratory insufficiency, that may manifest either during sleep or wakefulness.

The respiratory therapy device 122 is generally used to generate pressurized air that is delivered to a user (e.g., using one or more motors (such as a blower motor) that drive one or more compressors). In some implementations, the respiratory therapy device 122 generates continuous constant air pressure that is delivered to the user. In other implementations, the respiratory therapy device 122 generates two or more predetermined pressures (e.g., a first predetermined air pressure and a second predetermined air pressure). In still other implementations, the respiratory therapy device 122 is configured to generate a variety of different air pressures within a predetermined range. For example, the respiratory therapy device 122 can deliver at least about 6 cm H₂O, at least about 10 cm H₂O, at least about 20 cm H₂O, between about 6 cm H₂O and about 10 cm H₂O, between about 7 cm H₂O and about 12 cm H₂O, etc. The respiratory therapy device 122 can also deliver pressurized air at a predetermined flow rate between, for example, about -20 L/min and about 150 L/min, while maintaining a positive pressure (relative to the ambient pressure). In some implementations, the control system 110, the memory device 114, the electronic interface 119, or any combination thereof can be coupled to and/or positioned within a housing of the respiratory therapy device 122.

The user interface 124 engages a portion of the user's face and delivers pressurized air from the respiratory therapy device 122 to the user's airway to aid in preventing the airway from narrowing and/or collapsing during sleep. This may also increase the user's oxygen intake during sleep. Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 cm H₂O relative to ambient pressure. For other forms of therapy, such as the delivery of oxygen, the user interface may not include a seal sufficient to facilitate delivery to the airways of a supply of gas at a positive pressure of about 10 cmH₂O.

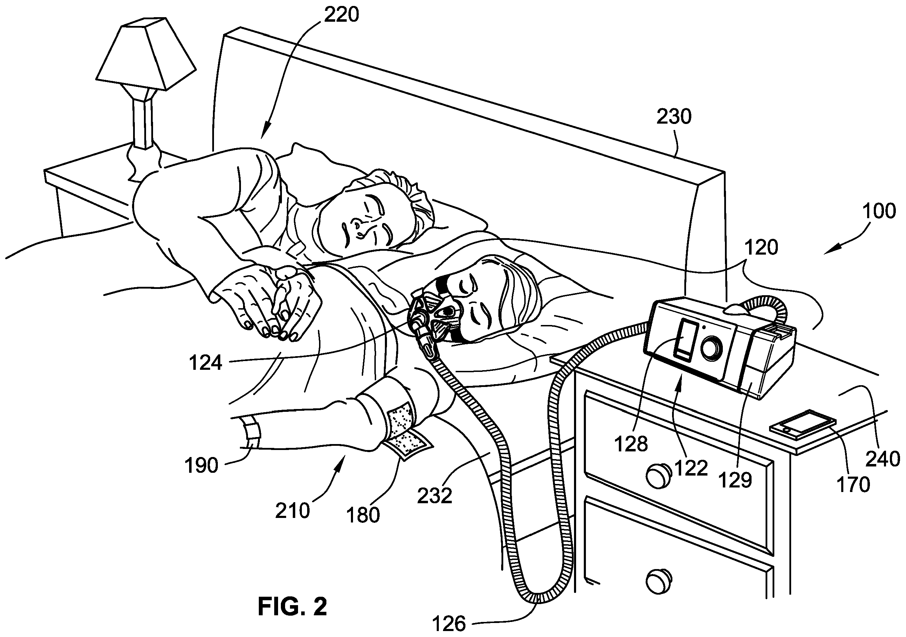

In some implementations, the user interface 124 is or includes a facial mask that covers the nose and mouth of the user (as shown, for example, in FIG. 2). Alternatively, the user interface 124 is or includes a nasal mask that provides air to the nose of the user or a nasal pillow mask that delivers air directly to the nostrils of the user. The user interface 124 can include a strap assembly that has a plurality of straps (e.g., including hook and loop fasteners) for positioning and/or stabilizing the user interface 124 on a portion of the user interface 124 on a desired location of the user (e.g., the face), and a conformal cushion (e.g., silicone, plastic, foam, etc.) that aids in providing an air-tight seal between the user interface 124 and the user. In some implementations, the user interface 124 may include a connector 127 and one or more vents 125. The one or more vents 125 can be used to permit the escape of carbon dioxide and other gases exhaled by the user. In other implementations, the user interface 124 includes a mouthpiece (e.g., a night guard mouthpiece molded to conform to the user's teeth, a mandibular repositioning device, etc.). In some implementations, the connector 127 is distinct from, but couplable to, the user interface 124 (and/or conduit 126). The connector 127 is configured to connect and fluidly couple the user interface 124 to the conduit 126.

The conduit 126 allows the flow of air between two components of a respiratory therapy system 120, such as the respiratory therapy device 122 and the user interface 124. In some implementations, there can be separate limbs of the conduit for inhalation and exhalation. In other implementations, a single limb conduit is used for both inhalation and exhalation. Generally, the respiratory therapy system 120 forms an air pathway that extends between a motor of the respiratory therapy device 122 and the user and/or the user's airway. Thus, the air pathway generally includes at least a motor of the respiratory therapy device 122, the user interface 124, and the conduit 126.

One or more of the respiratory therapy device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be used, for example, to measure the air pressure and/or flow rate of pressurized air supplied by the respiratory therapy device 122.

The display device 128 is generally used to display image(s) including still images, video images, or both and/or information regarding the respiratory therapy device 122. For example, the display device 128 can provide information regarding the status of the respiratory therapy device 122 (e.g., whether the respiratory therapy device 122 is on/off, the pressure of the air being delivered by the respiratory therapy device 122, the temperature of the air being delivered by the respiratory therapy device 122, etc.) and/or other information (e.g., a sleep score or a therapy score (such as a myAir^{®} score, such as described in WO 2016/061629 and US 2017/0311879), the current date/time, personal information for the user, a questionnaire for the user, etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory therapy device 122.

The humidification tank 129 is coupled to or integrated in the respiratory therapy device 122 and includes a reservoir of water that can be used to humidify the pressurized air delivered from the respiratory therapy device 122. The respiratory therapy device 122 can include a heater to heat the water in the humidification tank 129 in order to humidify the pressurized air provided to the user. Additionally, in some implementations, the conduit 126 can also include a heating element (e.g., coupled to and/or imbedded in the conduit 126) that heats the pressurized air delivered to the user. The humidification tank 129 can be fluidly coupled to a water vapor inlet of the air pathway and deliver water vapor into the air pathway via the water vapor inlet, or can be formed in-line with the air pathway as part of the air pathway itself. In other implementations, the respiratory therapy device 122 or the conduit 126 can include a waterless humidifier. The waterless humidifier can incorporate sensors that interface with other sensor positioned elsewhere in system 100.

The respiratory therapy system 120 can be used, for example, as a ventilator or a positive airway pressure (PAP) system, such as a continuous positive airway pressure (CPAP) system, an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based at least in part on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Referring to FIG. 2, a portion of the system 100 (FIG. 1), according to some implementations, is illustrated. A user 210 of the respiratory therapy system 120 and a bed partner 220 are located in a bed 230 and are laying on a mattress 232. The user interface 124 (e.g., a full facial mask) can be worn by the user 210 during a sleep session. The user interface 124 is fluidly coupled and/or connected to the respiratory therapy device 122 via the conduit 126. In turn, the respiratory therapy device 122 delivers pressurized air to the user 210 via the conduit 126 and the user interface 124 to increase the air pressure in the throat of the user 210 to aid in preventing the airway from closing and/or narrowing during sleep. The respiratory therapy device 122 can include the display device 128, which can allow the user to interact with the respiratory therapy device 122. The respiratory therapy device 122 can also include the humidification tank 129, which stores the water used to humidify the pressurized air. The respiratory therapy device 122 can be positioned on a nightstand 240 that is directly adjacent to the bed 230 as shown in FIG. 2, or more generally, on any surface or structure that is generally adjacent to the bed 230 and/or the user 210. The user can also wear the blood pressure device 180 and the activity tracker 190 while lying on the mattress 232 in the bed 230.

Referring back to FIG. 1, the one or more sensors 130 of the system 100 include a pressure sensor 132, a flow rate sensor 134, temperature sensor 136, a motion sensor 138, a microphone 140, a speaker 142, a radio-frequency (RF) receiver 146, an RF transmitter 148, a camera 150, an infrared (IR) sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a light detection and ranging (LiDAR) sensor 178, or any combination thereof. Generally, each of the one or sensors 130 are configured to output sensor data that is received and stored in the memory device 114 or one or more other memory devices. The sensors 130 can also include, an electrooculography (EOG) sensor, a peripheral oxygen saturation (SpO₂) sensor, a galvanic skin response (GSR) sensor, a carbon dioxide (CO₂) sensor, or any combination thereof.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the IR sensor 152, the PPG sensor 154, the ECG sensor 156, the EEG sensor 158, the capacitive sensor 160, the force sensor 162, the strain gauge sensor 164, the EMG sensor 166, the oxygen sensor 168, the analyte sensor 174, the moisture sensor 176, and the LiDAR sensor 178, more generally, the one or more sensors 130 can include any combination and any number of each of the sensors described and/or shown herein.

The one or more sensors 130 can be used to generate, for example physiological data, acoustic data, or both, that is associated with a user of the respiratory therapy system 120 (such as the user 210 of FIG. 2), the respiratory therapy system 120, both the user and the respiratory therapy system 120, or other entities, objects, activities, etc. Physiological data generated by one or more of the sensors 130 can be used by the control system 110 to determine a sleep-wake signal associated with the user during the sleep session and one or more sleep-related parameters. The sleep-wake signal can be indicative of one or more sleep stages (sometimes referred to as sleep states), including sleep, wakefulness, relaxed wakefulness, microawakenings, or distinct sleep stages such as a rapid eye movement (REM) stage (which can include both a typical REM stage and an atypical REM stage), a first non-REM stage (often referred to as "N1"), a second non-REM stage (often referred to as "N2"), a third non-REM stage (often referred to as "N3"), or any combination thereof. Methods for determining sleep stages from physiological data generated by one or more of the sensors, such as sensors 130, are described in, for example, WO 2014/047310, US 10,492,720, US 10,660,563, US 2020/0337634, WO 2017/132726, WO 2019/122413, US 2021/0150873, WO 2019/122414, US 2020/0383580.

The sleep-wake signal can also be timestamped to indicate a time that the user enters the bed, a time that the user exits the bed, a time that the user attempts to fall asleep, etc. The sleep-wake signal can be measured one or more of the sensors 130 during the sleep session at a predetermined sampling rate, such as, for example, one sample per second, one sample per 30 seconds, one sample per minute, etc. Examples of the one or more sleep-related parameters that can be determined for the user during the sleep session based at least in part on the sleep-wake signal include a total time in bed, a total sleep time, a total wake time, a sleep onset latency, a wake-after-sleep-onset parameter, a sleep efficiency, a fragmentation index, an amount of time to fall asleep, a consistency of breathing rate, a fall asleep time, a wake time, a rate of sleep disturbances, a number of movements, or any combination thereof.

Physiological data and/or acoustic data generated by the one or more sensors 130 can also be used to determine a respiration signal associated with the user during a sleep session. The respiration signal is generally indicative of respiration or breathing of the user during the sleep session. The respiration signal can be indicative of, for example, a respiration rate, a respiration rate variability, an inspiration amplitude, an expiration amplitude, an inspiration-expiration amplitude ratio, an inspiration-expiration duration ratio, a number of events per hour, a pattern of events, pressure settings of the respiratory therapy device 122, or any combination thereof. The event(s) can include snoring, apneas, central apneas, obstructive apneas, mixed apneas, hypopneas, RERAs, a flow limitation (e.g., an event that results in the absence of the increase in flow despite an elevation in negative intrathoracic pressure indicating increased effort), a mask leak (e.g., from the user interface 124), a restless leg, a sleeping disorder, choking, an increased heart rate, a heart rate variation, labored breathing, an asthma attack, an epileptic episode, a seizure, a fever, a cough, a sneeze, a snore, a gasp, the presence of an illness such as the common cold or the flu, an elevated stress level, etc. Events can be detected by any means known in the art such as described in, for example, US 5,245,995, US 6,502,572, WO 2018/050913, WO 2020/104465.

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory therapy system 120 and/or ambient pressure. In such implementations, the pressure sensor 132 can be coupled to or integrated in the respiratory therapy device 122. The pressure sensor 132 can be, for example, a capacitive sensor, an electromagnetic sensor, an inductive sensor, a resistive sensor, a piezoelectric sensor, a strain-gauge sensor, an optical sensor, a potentiometric sensor, or any combination thereof. In one example, the pressure sensor 132 can be used to determine a blood pressure of the user.

The flow rate sensor 134 outputs flow rate data that can be stored in the memory device 114 and/or analyzed by the processor 112 of the control system 110. In some implementations, the flow rate sensor 134 is used to determine an air flow rate from the respiratory therapy device 122, an air flow rate through the conduit 126, an air flow rate through the user interface 124, or any combination thereof. In such implementations, the flow rate sensor 134 can be coupled to or integrated in the respiratory therapy device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof.

The temperature sensor 136 outputs temperature data that can be stored in the memory device 114 and/or analyzed by the processor 112 of the control system 110. In some implementations, the temperature sensor 136 generates temperatures data indicative of a core body temperature of the user, a skin temperature of the user 210, a temperature of the air flowing from the respiratory therapy device 122 and/or through the conduit 126, a temperature in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The motion sensor 138 outputs motion data that can be stored in the memory device 114 and/or analyzed by the processor 112 of the control system 110. The motion sensor 138 can be used to detect movement of the user during the sleep session, and/or detect movement of any of the components of the respiratory therapy system 120, such as the respiratory therapy device 122, the user interface 124, or the conduit 126. The motion sensor 138 can include one or more inertial sensors, such as accelerometers, gyroscopes, and magnetometers. The motion sensor 138 can be used to detect motion or acceleration associated with arterial pulses, such as pulses in or around the face of the user and proximal to the user interface 124, and configured to detect features of the pulse shape, speed, amplitude, or volume. In some implementations, the motion sensor 138 alternatively or additionally generates one or more signals representing bodily movement of the user, from which may be obtained a signal representing a sleep state of the user; for example, via a respiratory movement of the user.

The microphone 140 outputs acoustic data that can be stored in the memory device 114 and/or analyzed by the processor 112 of the control system 110. The acoustic data generated by the microphone 140 is reproducible as one or more sound(s) during a sleep session (e.g., sounds from the user) to determine (e.g., using the control system 110) one or more sleep-related parameters, as described in further detail herein. The acoustic data from the microphone 140 can also be used to identify (e.g., using the control system 110) an event experienced by the user during the sleep session, as described in further detail herein. In other implementations, the acoustic data from the microphone 140 is representative of noise associated with the respiratory therapy system 120. In some implementations, the system 100 includes a plurality of microphones (e.g., two or more microphones and/or an array of microphones with beamforming) such that sound data generated by each of the plurality of microphones can be used to discriminate the sound data generated by another of the plurality of microphones. The microphone 140 can be coupled to or integrated in the respiratory therapy system 120 (or the system 100) generally in any configuration. For example, the microphone 140 can be disposed inside the respiratory therapy device 122, the user interface 124, the conduit 126, or other components. The microphone 140 can also be positioned adjacent to or coupled to the outside of the respiratory therapy device 122, the outside of the user interface 124, the outside of the conduit 126, or outside of any other components. The microphone 140 could also be a component of the user device 170 (e.g., the microphone 140 is a microphone of a smart phone). The microphone 140 can be integrated into the user interface 124, the conduit 126, the respiratory therapy device 122, or any combination thereof. In general, the microphone 140 can be located at any point within or adjacent to the air pathway of the respiratory therapy system 120, which includes at least the motor of the respiratory therapy device 122, the user interface 124, and the conduit 126. Thus, the air pathway can also be referred to as the acoustic pathway.

The speaker 142 outputs sound waves that are typically audible to the user. In one or more implementations, the sound waves can be audible to a user of the system 100 or inaudible to the user of the system (e.g., ultrasonic sound waves). The speaker 142 can be used, for example, as an alarm clock or to play an alert or message to the user (e.g., in response to an event). In some implementations, the speaker 142 can be used to communicate the acoustic data generated by the microphone 140 to the user. The speaker 142 can be coupled to or integrated in the respiratory therapy device 122, the user interface 124, the conduit 126, or the user device 170.

The microphone 140 and the speaker 142 can be used as separate devices. In some implementations, the microphone 140 and the speaker 142 can be combined into an acoustic sensor 141 (e.g., a SONAR sensor), as described in, for example, WO 2018/050913 and WO 2020/104465. In such implementations, the speaker 142 generates or emits sound waves at a predetermined interval and/or frequency, and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user or a bed partner of the user (such as bed partner 220 in FIG. 2). Based at least in part on the data from the microphone 140 and/or the speaker 142, the control system 110 can determine a location of the user and/or one or more of the sleep-related parameters described in herein, such as, for example, a respiration signal, a respiration rate, an inspiration amplitude, an expiration amplitude, an inspiration-expiration ratio, a number of events per hour, a pattern of events, a sleep stage, pressure settings of the respiratory therapy device 122, a mouth leak status, or any combination thereof. In this context, a SONAR sensor may be understood to concern an active acoustic sensing, such as by generating/transmitting ultrasound or low frequency ultrasound sensing signals (e.g., in a frequency range of about 17-23 kHz, 18-22 kHz, or 17-18 kHz, for example), through the air. Such a system may be considered in relation to WO 2018/050913 and WO 2020/104465 mentioned above. In some implementations, the speaker 142 is a bone conduction speaker. In some implementations, the one or more sensors 130 include (i) a first microphone that is the same or similar to the microphone 140, and is integrated into the acoustic sensor 141 and (ii) a second microphone that is the same as or similar to the microphone 140, but is separate and distinct from the first microphone that is integrated into the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location of the user and/or one or more of the sleep-related parameters described herein. An RF receiver (either the RF receiver 146 and the RF transmitter 148 or another RF pair) can also be used for wireless communication between the control system 110, the respiratory therapy device 122, the one or more sensors 130, the user device 170, or any combination thereof. While the RF receiver 146 and RF transmitter 148 are shown as being separate and distinct elements in FIG. 1, in some implementations, the RF receiver 146 and RF transmitter 148 are combined as a part of an RF sensor 147 (e.g., a RADAR sensor). In some such implementations, the RF sensor 147 includes a control circuit. The specific format of the RF communication could be WiFi, Bluetooth, etc.

In some implementations, the RF sensor 147 is a part of a mesh system. One example of a mesh system is a WiFi mesh system, which can include mesh nodes, mesh router(s), and mesh gateway(s), each of which can be mobile/movable or fixed. In such implementations, the WiFi mesh system includes a WiFi router and/or a WiFi controller and one or more satellites (e.g., access points), each of which include an RF sensor that the is the same as, or similar to, the RF sensor 147. The WiFi router and satellites continuously communicate with one another using WiFi signals. The WiFi mesh system can be used to generate motion data based at least in part on changes in the WiFi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or a combination thereof) that can be stored in the memory device 114. The image data from the camera 150 can be used by the control system 110 to determine one or more of the sleep-related parameters described herein. For example, the image data from the camera 150 can be used to identify a location of the user, to determine a time when the user enters the user's bed (such as bed 230 in FIG. 2), and to determine a time when the user exits the bed 230. The camera 150 can also be used to track eye movements, pupil dilation (if one or both of the user's eyes are open), blink rate, or any changes during REM sleep. The camera 150 can also be used to track the position of the user, which can impact the duration and/or severity of apneic episodes in users with positional obstructive sleep apnea.

The IR sensor 152 outputs infrared image data reproducible as one or more infrared images (e.g., still images, video images, or both) that can be stored in the memory device 114. The infrared data from the IR sensor 152 can be used to determine one or more sleep-related parameters during the sleep session, including a temperature of the user and/or movement of the user. The IR sensor 152 can also be used in conjunction with the camera 150 when measuring the presence, location, and/or movement of the user. The IR sensor 152 can detect infrared light having a wavelength between about 700 nm and about 1 mm, for example, while the camera 150 can detect visible light having a wavelength between about 380 nm and about 740 nm.

The IR sensor 152 outputs infrared image data reproducible as one or more infrared images (e.g., still images, video images, or both) that can be stored in the memory device 114. The infrared data from the IR sensor 152 can be used to determine one or more sleep-related parameters during the sleep session, including a temperature of the user and/or movement of the user. The IR sensor 152 can also be used in conjunction with the camera 150 when measuring the presence, location, and/or movement of the user. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user that can be used to determine one or more sleep-related parameters, such as, for example, a heart rate, a heart rate pattern, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user, embedded in clothing and/or fabric that is worn by the user, embedded in and/or coupled to the user interface 124 and/or its associated headgear (e.g., straps, etc.), etc.

The ECG sensor 156 outputs physiological data associated with electrical activity of the heart of the user. In some implementations, the ECG sensor 156 includes one or more electrodes that are positioned on or around a portion of the user during the sleep session. The physiological data from the ECG sensor 156 can be used, for example, to determine one or more of the sleep-related parameters described herein.

The EEG sensor 158 outputs physiological data associated with electrical activity of the brain of the user. In some implementations, the EEG sensor 158 includes one or more electrodes that are positioned on or around the scalp of the user during the sleep session. The physiological data from the EEG sensor 158 can be used, for example, to determine a sleep stage of the user at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the exhaled breath of the user. The data output by the analyte sensor 174 can be stored in the memory device 114 and used by the control system 110 to determine the identity and concentration of any analytes in the user's breath. In some implementations, the analyte sensor 174 is positioned near a mouth of the user to detect analytes in breath exhaled from the user's mouth. For example, when the user interface 124 is a facial mask that covers the nose and mouth of the user, the analyte sensor 174 can be positioned within the facial mask to monitor the user mouth breathing. In other implementations, such as when the user interface 124 is a nasal mask or a nasal pillow mask, the analyte sensor 174 can be positioned near the nose of the user to detect analytes in breath exhaled through the user's nose. In still other implementations, the analyte sensor 174 can be positioned near the user's mouth when the user interface 124 is a nasal mask or a nasal pillow mask. In this implementation, the analyte sensor 174 can be used to detect whether any air is inadvertently leaking from the user's mouth. In some implementations, the analyte sensor 174 is a volatile organic compound (VOC) sensor that can be used to detect carbon-based chemicals or compounds, such as carbon dioxide. In some implementations, the analyte sensor 174 can also be used to detect whether the user is breathing through their nose or mouth. For example, if the data output by an analyte sensor 174 positioned near the mouth of the user or within the facial mask (in implementations where the user interface 124 is a facial mask) detects the presence of an analyte, the control system 110 can use this data as an indication that the user is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user's face, near the connection between the conduit 126 and the user interface 124, near the connection between the conduit 126 and the respiratory therapy device 122, etc.). Thus, in some implementations, the moisture sensor 176 can be coupled to or integrated into the user interface 124 or in the conduit 126 to monitor the humidity of the pressurized air from the respiratory therapy device 122. In other implementations, the moisture sensor 176 is placed near any area where moisture levels need to be monitored. The moisture sensor 176 can also be used to monitor the humidity of the ambient environment surrounding the user, for example the air inside the user's bedroom. The moisture sensor 176 can also be used to track the user's biometric response to environmental changes.

One or more LiDAR sensors 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 178 can measure and map an area extending 5 meters or more away from the sensor. The LiDAR data can be fused with point cloud data estimated by an electromagnetic RADAR sensor, for example. The LiDAR sensor 178 may also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

While shown separately in FIG. 1, any combination of the one or more sensors 130 can be integrated in and/or coupled to any one or more of the components of the system 100, including the respiratory therapy device 122, the user interface 124, the conduit 126, the humidification tank 129, the control system 110, the user device 170, or any combination thereof. For example, the acoustic sensor 141 and/or the RF sensor 147 can be integrated in and/or coupled to the user device 170. In such implementations, the user device 170 can be considered a secondary device that generates additional or secondary data for use by the system 100 (e.g., the control system 110) according to some aspects of the present disclosure. In some implementations, the pressure sensor 132 and/or the flow rate sensor 134 are integrated into and/or coupled to the respiratory therapy device 122. In some implementations, at least one of the one or more sensors 130 is not coupled to the respiratory therapy device 122, the control system 110, or the user device 170, and is positioned generally adjacent to the user during the sleep session (e.g., positioned on or in contact with a portion of the user, worn by the user, coupled to or positioned on the nightstand, coupled to the mattress, coupled to the ceiling, etc.). More generally, the one or more sensors 130 can be positioned at any suitable location relative to the user such that the one or more sensors 130 can generate physiological data associated with the user and/or the bed partner 220 during one or more sleep session.

The data from the one or more sensors 130 can be analyzed to determine one or more sleep-related parameters, which can include a respiration signal, a respiration rate, a respiration pattern, an inspiration amplitude, an expiration amplitude, an inspiration-expiration ratio, an occurrence of one or more events, a number of events per hour, a pattern of events, an average duration of events, a range of event durations, a ratio between the number of different events, a sleep stage, an apnea-hypopnea index (AHI), or any combination thereof. The one or more events can include snoring, apneas, central apneas, obstructive apneas, mixed apneas, hypopneas, an intentional user interface leak, an unintentional user interface leak, a mouth leak, a cough, a restless leg, a sleeping disorder, choking, an increased heart rate, labored breathing, an asthma attack, an epileptic episode, a seizure, increased blood pressure, hyperventilation, or any combination thereof. Many of these sleep-related parameters are physiological parameters, although some of the sleep-related parameters can be considered to be non-physiological parameters. Other types of physiological and non-physiological parameters can also be determined, either from the data from the one or more sensors 130, or from other types of data.

The user device 170 includes a display device 172. The user device 170 can be, for example, a mobile device such as a smart phone, a tablet, a laptop, a gaming console, a smart watch, or the like. Alternatively, the user device 170 can be an external sensing system, a television (e.g., a smart television) or another smart home device (e.g., a smart speaker(s) such as Google Home^{®}, Google Nest^{®}, Amazon Echo^{®}, Amazon Echo Show^{®}, Alexa^{®}-enabled devices, etc.). In some implementations, the user device 170 is a wearable device (e.g., a smart watch). The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the user device 170. In some implementations, one or more user devices 170 can be used by and/or included in the system 100.

The blood pressure device 180 is generally used to aid in generating physiological data for determining one or more blood pressure measurements associated with a user. The blood pressure device 180 can include at least one of the one or more sensors 130 to measure, for example, a systolic blood pressure component and/or a diastolic blood pressure component.

In some implementations, the blood pressure device 180 is a sphygmomanometer including an inflatable cuff that can be worn by a user and a pressure sensor (e.g., the pressure sensor 132 described herein). For example, as shown in the example of FIG. 2, the blood pressure device 180 can be worn on an upper arm of the user. In such implementations where the blood pressure device 180 is a sphygmomanometer, the blood pressure device 180 also includes a pump (e.g., a manually operated bulb) for inflating the cuff. In some implementations, the blood pressure device 180 is coupled to the respiratory therapy device 122 of the respiratory therapy system 120, which in turn delivers pressurized air to inflate the cuff. More generally, the blood pressure device 180 can be communicatively coupled with, and/or physically integrated in (e.g., within a housing), the control system 110, the memory device 114, the respiratory therapy system 120, the user device 170, and/or the activity tracker 190.

The activity tracker 190 is generally used to aid in generating physiological data for determining an activity measurement associated with the user. The activity measurement can include, for example, a number of steps, a distance traveled, a number of steps climbed, a duration of physical activity, a type of physical activity, an intensity of physical activity, time spent standing, a respiration rate, an average respiration rate, a resting respiration rate, a maximum respiration rate, a respiration rate variability, a heart rate, an average heart rate, a resting heart rate, a maximum heart rate, a heart rate variability, a number of calories burned, blood oxygen saturation, electrodermal activity (also known as skin conductance or galvanic skin response), or any combination thereof. The activity tracker 190 includes one or more of the sensors 130 described herein, such as, for example, the motion sensor 138 (e.g., one or more accelerometers and/or gyroscopes), the PPG sensor 154, and/or the ECG sensor 156.

In some implementations, the activity tracker 190 is a wearable device that can be worn by the user, such as a smartwatch, a wristband, a ring, or a patch. For example, referring to FIG. 2, the activity tracker 190 is worn on a wrist of the user. The activity tracker 190 can also be coupled to or integrated a garment or clothing that is worn by the user. Alternatively, still, the activity tracker 190 can also be coupled to or integrated in (e.g., within the same housing) the user device 170. More generally, the activity tracker 190 can be communicatively coupled with, or physically integrated in (e.g., within a housing), the control system 110, the memory device 114, the respiratory therapy system 120, the user device 170, and/or the blood pressure device 180.

While the control system 110 and the memory device 114 are described and shown in FIG. 1 as being a separate and distinct component of the system 100, in some implementations, the control system 110 and/or the memory device 114 are integrated in the user device 170 and/or the respiratory therapy device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

While system 100 is shown as including all of the components described above, more or fewer components can be included in a system for identifying a user interface used by a user, according to implementations of the present disclosure. For example, a first alternative system includes the control system 110, the memory device 114, and at least one of the one or more sensors 130. As another example, a second alternative system includes the control system 110, the memory device 114, at least one of the one or more sensors 130, and the user device 170. As yet another example, a third alternative system includes the control system 110, the memory device 114, the respiratory therapy system 120, at least one of the one or more sensors 130, and the user device 170. As a further example, a fourth alternative system includes the control system 110, the memory device 114, the respiratory therapy system 120, at least one of the one or more sensors 130, the user device 170, and the blood pressure device 180 and/or activity tracker 190. Thus, various systems for modifying pressure settings can be formed using any portion or portions of the components shown and described herein and/or in combination with one or more other components.

Referring again to FIG. 2, in some implementations, the control system 110, the memory device 114, any of the one or more sensors 130, or a combination thereof can be located on and/or in any surface and/or structure that is generally adjacent to the bed 230 and/or the user 210. For example, in some implementations, at least one of the one or more sensors 130 can be located at a first position on and/or in one or more components of the respiratory therapy system 120 adjacent to the bed 230 and/or the user 210. The one or more sensors 130 can be coupled to the respiratory therapy system 120, the user interface 124, the conduit 126, the display device 128, the humidification tank 129, or a combination thereof.

Alternatively, or additionally, at least one of the one or more sensors 130 can be located at a second position on and/or in the bed 230 (e.g., the one or more sensors 130 are coupled to and/or integrated in the bed 230). Further, alternatively or additionally, at least one of the one or more sensors 130 can be located at a third position on and/or in the mattress 232 that is adjacent to the bed 230 and/or the user 210 (e.g., the one or more sensors 130 are coupled to and/or integrated in the mattress 232). Alternatively, or additionally, at least one of the one or more sensors 130 can be located at a fourth position on and/or in a pillow that is generally adjacent to the bed 230 and/or the user 210.

Alternatively, or additionally, at least one of the one or more sensors 130 can be located at a fifth position on and/or in the nightstand 240 that is generally adjacent to the bed 230 and/or the user 210. Alternatively, or additionally, at least one of the one or more sensors 130 can be located at a sixth position such that the at least one of the one or more sensors 130 are coupled to and/or positioned on the user 210 (e.g., the one or more sensors 130 are embedded in or coupled to fabric, clothing, and/or a smart device worn by the user 210). More generally, at least one of the one or more sensors 130 can be positioned at any suitable location relative to the user 210 such that the one or more sensors 130 can generate sensor data associated with the user 210.

In some implementations, a primary sensor, such as the microphone 140, is configured to generate acoustic data associated with the user 210 during a sleep session. For example, one or more microphones (the same as, or similar to, the microphone 140 of FIG. 1) can be integrated in and/or coupled to (i) a circuit board of the respiratory therapy device 122, (ii) the conduit 126, (iii) a connector between components of the respiratory therapy system 120, (iv) the user interface 124, (v) a headgear (e.g., straps) associated with the user interface, or (vi) a combination thereof.

In some implementations, one or more secondary sensors may be used in addition to the primary sensor to generate additional data. In some such implementations, the one or more secondary sensors include: a microphone (e.g., the microphone 140 of the system 100), a flow rate sensor (e.g., the flow rate sensor 134 of the system 100), a pressure sensor (e.g., the pressure sensor 132 of the system 100), a temperature sensor (e.g., the temperature sensor 136 of the system 100), a camera (e.g., the camera 150 of the system 100), a vane sensor (VAF), a hot wire sensor (MAF), a cold wire sensor, a laminar flow sensor, an ultrasonic sensor, an inertial sensor, or a combination thereof.

Additionally, or alternatively, one or more microphones (the same as, or similar to, the microphone 140 of FIG. 1) can be integrated in and/or coupled to a co-located smart device, such as the user device 170, a TV, a watch (e.g., a mechanical watch or another smart device worn by the user), a pendant, the mattress 232, the bed 230, beddings positioned on the bed 230, the pillow, a speaker (e.g., the speaker 142 of FIG. 1), a radio, a tablet device, a waterless humidifier, or a combination thereof. A co-located smart device can be any smart device that is within range for detecting sounds emitted by the user, the respiratory therapy system 120, and/or any portion of the system 100. In some implementations, the co-located smart device is a smart device that is in the same room as the user during the sleep session.

Additionally, or alternatively, in some implementations, one or more microphones (the same as, or similar to, the microphone 140 of FIG. 1) can be remote from the system 100 (FIG. 1) and/or the user 210 (FIG. 2), so long as there is an air passage allowing acoustic signals to travel to the one or more microphones. For example, the one or more microphones can be in a different room from the room containing the system 100.

As used herein, a sleep session can be defined in a number of ways based at least in part on, for example, an initial start time and an end time. In some implementations, a sleep session is a duration where the user is asleep, that is, the sleep session has a start time and an end time, and during the sleep session, the user does not wake until the end time. That is, any period of the user being awake is not included in a sleep session. From this first definition of sleep session, if the user wakes ups and falls asleep multiple times in the same night, each of the sleep intervals separated by an awake interval is a sleep session.

Alternatively, in some implementations, a sleep session has a start time and an end time, and during the sleep session, the user can wake up, without the sleep session ending, so long as a continuous duration that the user is awake is below an awake duration threshold. The awake duration threshold can be defined as a percentage of a sleep session. The awake duration threshold can be, for example, about twenty percent of the sleep session, about fifteen percent of the sleep session duration, about ten percent of the sleep session duration, about five percent of the sleep session duration, about two percent of the sleep session duration, etc., or any other threshold percentage. In some implementations, the awake duration threshold is defined as a fixed amount of time, such as, for example, about one hour, about thirty minutes, about fifteen minutes, about ten minutes, about five minutes, about two minutes, etc., or any other amount of time.

In some implementations, a sleep session is defined as the entire time between the time in the evening at which the user first entered the bed, and the time the next morning when user last left the bed. Put another way, a sleep session can be defined as a period of time that begins on a first date (e.g., Monday, January 6, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, January 7, 2020) at a second time (e.g., 7:00 AM), that can be referred to as the next morning, when the user first exits the bed with the intention of not going back to sleep that next morning.

In some implementations, the user can manually define the beginning of a sleep session and/or manually terminate a sleep session. For example, the user can select (e.g., by clicking or tapping) one or more user-selectable element that is displayed on the display device 172 of the user device 170 (FIG. 1) to manually initiate or terminate the sleep session.

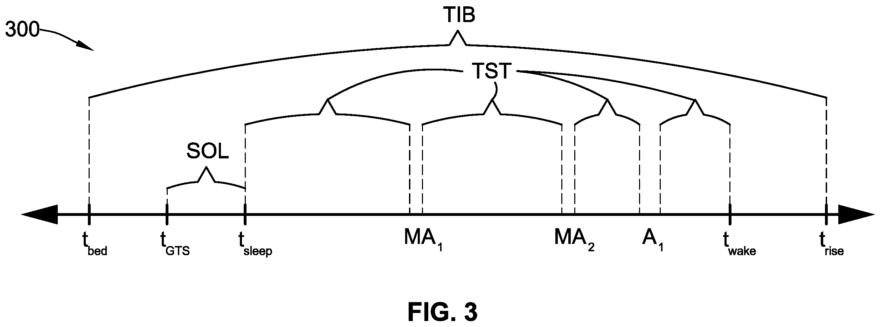

Referring to FIG. 3, an exemplary timeline 300 for a sleep session is illustrated. The timeline 300 includes an enter bed time (t_{bed}), a go-to-sleep time (t_{GTS}), an initial sleep time (tₛₗₑₑₚ), a first micro-awakening MA₁, a second micro-awakening MA₂, an awakening A, a wake-up time (t_{wake}), and a rising time (tᵣᵢₛₑ).

The enter bed time t_{bed} is associated with the time that the user initially enters the bed (e.g., bed 230 in FIG. 2) prior to falling asleep (e.g., when the user lies down or sits in the bed). The enter bed time t_{bed} can be identified based at least in part on a bed threshold duration to distinguish between times when the user enters the bed for sleep and when the user enters the bed for other reasons (e.g., to watch TV). For example, the bed threshold duration can be at least about 10 minutes, at least about 20 minutes, at least about 30 minutes, at least about 45 minutes, at least about 1 hour, at least about 2 hours, etc. While the enter bed time t_{bed} is described herein in reference to a bed, more generally, the enter time t_{bed} can refer to the time the user initially enters any location for sleeping (e.g., a couch, a chair, a sleeping bag, etc.).

The go-to-sleep time (GTS) is associated with the time that the user initially attempts to fall asleep after entering the bed (t_{bed}). For example, after entering the bed, the user may engage in one or more activities to wind down prior to trying to sleep (e.g., reading, watching TV, listening to music, using the user device 170, etc.). The initial sleep time (tₛₗₑₑₚ) is the time that the user initially falls asleep. For example, the initial sleep time (tₛₗₑₑₚ) can be the time that the user initially enters the first non-REM sleep stage.

The wake-up time t_{wake} is the time associated with the time when the user wakes up without going back to sleep (e.g., as opposed to the user waking up in the middle of the night and going back to sleep). The user may experience one of more unconscious microawakenings (e.g., microawakenings MA₁ and MA₂) having a short duration (e.g., 5 seconds, 10 seconds, 30 seconds, 1 minute, etc.) after initially falling asleep. In contrast to the wake-up time t_{wake}, the user goes back to sleep after each of the microawakenings MA₁ and MA₂. Similarly, the user may have one or more conscious awakenings (e.g., awakening A) after initially falling asleep (e.g., getting up to go to the bathroom, attending to children or pets, sleep walking, etc.). However, the user goes back to sleep after the awakening A. Thus, the wake-up time t_{wake} can be defined, for example, based at least in part on a wake threshold duration (e.g., the user is awake for at least 15 minutes, at least 20 minutes, at least 30 minutes, at least 1 hour, etc.).

Similarly, the rising time tᵣᵢₛₑ is associated with the time when the user exits the bed and stays out of the bed with the intent to end the sleep session (e.g., as opposed to the user getting up during the night to go to the bathroom, to attend to children or pets, sleep walking, etc.). In other words, the rising time tᵣᵢₛₑ is the time when the user last leaves the bed without returning to the bed until a next sleep session (e.g., the following evening). Thus, the rising time tᵣᵢₛₑ can be defined, for example, based at least in part on a rise threshold duration (e.g., the user has left the bed for at least 15 minutes, at least 20 minutes, at least 30 minutes, at least 1 hour, etc.). The enter bed time t_{bed} time for a second, subsequent sleep session can also be defined based at least in part on a rise threshold duration (e.g., the user has left the bed for at least 4 hours, at least 6 hours, at least 8 hours, at least 12 hours, etc.).

As described above, the user may wake up and get out of bed one more times during the night between the initial t_{bed} and the final tᵣᵢₛₑ. In some implementations, the final wake-up time t_{wake} and/or the final rising time tᵣᵢₛₑ that are identified or determined based at least in part on a predetermined threshold duration of time subsequent to an event (e.g., falling asleep or leaving the bed). Such a threshold duration can be customized for the user. For a standard user which goes to bed in the evening, then wakes up and goes out of bed in the morning any period (between the user waking up (t_{wake}) or raising up (tᵣᵢₛₑ), and the user either going to bed (t_{bed}), going to sleep (T_{GTS}) or falling asleep (tₛₗₑₑₚ) of between about 12 and about 18 hours can be used. For users that spend longer periods of time in bed, shorter threshold periods may be used (e.g., between about 8 hours and about 14 hours). The threshold period may be initially selected and/or later adjusted based at least in part on the system monitoring the user's sleep behavior.

The total time in bed (TIB) is the duration of time between the time enter bed time t_{bed} and the rising time tᵣᵢₛₑ. The total sleep time (TST) is associated with the duration between the initial sleep time and the wake-up time, excluding any conscious or unconscious awakenings and/or micro-awakenings therebetween. Generally, the total sleep time (TST) will be shorter than the total time in bed (TIB) (e.g., one minute short, ten minutes shorter, one hour shorter, etc.). For example, referring to the timeline 300 of FIG. 3, the total sleep time (TST) spans between the initial sleep time tₛₗₑₑₚ and the wake-up time t_{wake}, but excludes the duration of the first micro-awakening MA₁, the second micro-awakening MA₂, and the awakening A. As shown, in this example, the total sleep time (TST) is shorter than the total time in bed (TIB).

In some implementations, the total sleep time (TST) can be defined as a persistent total sleep time (PTST). In such implementations, the persistent total sleep time excludes a predetermined initial portion or period of the first non-REM stage (e.g., light sleep stage). For example, the predetermined initial portion can be between about 30 seconds and about 20 minutes, between about 1 minute and about 10 minutes, between about 3 minutes and about 5 minutes, etc. The persistent total sleep time is a measure of sustained sleep, and smooths the sleep-wake hypnogram. For example, when the user is initially falling asleep, the user may be in the first non-REM stage for a very short time (e.g., about 30 seconds), then back into the wakefulness stage for a short period (e.g., one minute), and then goes back to the first non-REM stage. In this example, the persistent total sleep time excludes the first instance (e.g., about 30 seconds) of the first non-REM stage.

In some implementations, the sleep session is defined as starting at the enter bed time (t_{bed}) and ending at the rising time (tᵣᵢₛₑ), i.e., the sleep session is defined as the total time in bed (TIB). In some implementations, a sleep session is defined as starting at the initial sleep time (tₛₗₑₑₚ) and ending at the wake-up time (t_{wake}). In some implementations, the sleep session is defined as the total sleep time (TST). In some implementations, a sleep session is defined as starting at the go-to-sleep time (T_{GTS}) and ending at the wake-up time (t_{wake}). In some implementations, a sleep session is defined as starting at the go-to-sleep time (t_{GTS}) and ending at the rising time (tᵣᵢₛₑ). In some implementations, a sleep session is defined as starting at the enter bed time (t_{bed}) and ending at the wake-up time (t_{wake}). In some implementations, a sleep session is defined as starting at the initial sleep time (tₛₗₑₑₚ) and ending at the rising time (tᵣᵢₛₑ).

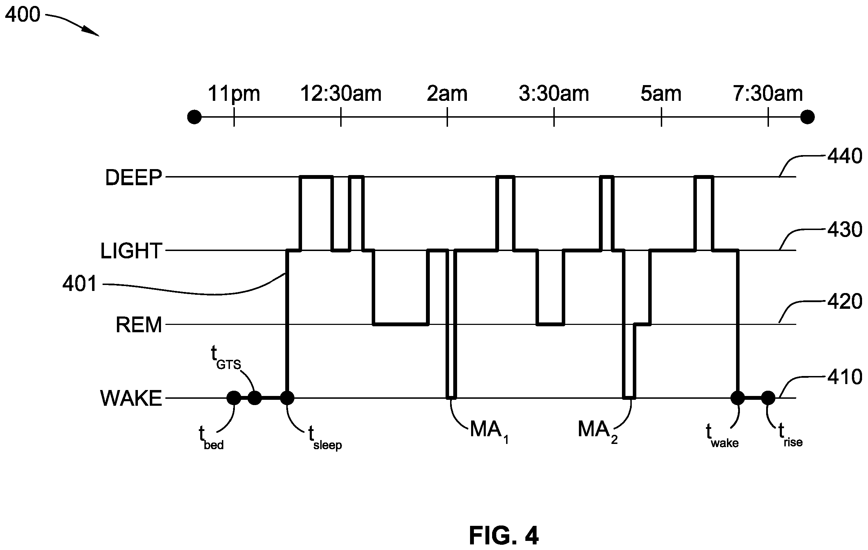

Referring to FIG. 4, an exemplary hypnogram 400 corresponding to the timeline 300 (FIG. 3), according to some implementations, is illustrated. As shown, the hypnogram 400includes a sleep-wake signal 401, a wakefulness stage axis 410, a REM stage axis 420, a light sleep stage axis 430, and a deep sleep stage axis 440. The intersection between the sleep-wake signal 401 and one of the axes 410-440 is indicative of the sleep stage at any given time during the sleep session.

The sleep-wake signal 401 can be generated based at least in part on physiological data associated with the user (e.g., generated by one or more of the sensors 130 described herein). The sleep-wake signal can be indicative of one or more sleep stages, including wakefulness, relaxed wakefulness, microawakenings, a REM stage, a first non-REM stage, a second non-REM stage, a third non-REM stage, or any combination thereof. In some implementations, one or more of the first non-REM stage, the second non-REM stage, and the third non-REM stage can be grouped together and categorized as a light sleep stage or a deep sleep stage. For example, the light sleep stage can include the first non-REM stage and the deep sleep stage can include the second non-REM stage and the third non-REM stage. While the hypnogram 400 is shown in FIG. 4 as including the light sleep stage axis 430 and the deep sleep stage axis 440, in some implementations, the hypnogram 400 can include an axis for each of the first non-REM stage, the second non-REM stage, and the third non-REM stage. In other implementations, the sleep-wake signal can also be indicative of a respiration signal, a respiration rate, an inspiration amplitude, an expiration amplitude, an inspiration-expiration amplitude ratio, an inspiration-expiration duration ratio, a number of events per hour, a pattern of events, or any combination thereof. Information describing the sleep-wake signal can be stored in the memory device 114.

The hypnogram 400 can be used to determine one or more sleep-related parameters, such as, for example, a sleep onset latency (SOL), wake-after-sleep onset (WASO), a sleep efficiency (SE), a sleep fragmentation index, sleep blocks, or any combination thereof.

The sleep onset latency (SOL) is defined as the time between the go-to-sleep time (T_{GTS}) and the initial sleep time (tₛₗₑₑₚ). In other words, the sleep onset latency is indicative of the time that it took the user to actually fall asleep after initially attempting to fall asleep. In some implementations, the sleep onset latency is defined as a persistent sleep onset latency (PSOL). The persistent sleep onset latency differs from the sleep onset latency in that the persistent sleep onset latency is defined as the duration time between the go-to-sleep time and a predetermined amount of sustained sleep. In some implementations, the predetermined amount of sustained sleep can include, for example, at least 10 minutes of sleep within the second non-REM stage, the third non-REM stage, and/or the REM stage with no more than 2 minutes of wakefulness, the first non-REM stage, and/or movement therebetween. In other words, the persistent sleep onset latency requires up to, for example, 8 minutes of sustained sleep within the second non-REM stage, the third non-REM stage, and/or the REM stage. In other implementations, the predetermined amount of sustained sleep can include at least 10 minutes of sleep within the first non-REM stage, the second non-REM stage, the third non-REM stage, and/or the REM stage subsequent to the initial sleep time. In such implementations, the predetermined amount of sustained sleep can exclude any micro-awakenings (e.g., a ten second micro-awakening does not restart the 10-minute period).

The wake-after-sleep onset (WASO) is associated with the total duration of time that the user is awake between the initial sleep time and the wake-up time. Thus, the wake-after-sleep onset includes short and micro-awakenings during the sleep session (e.g., the microawakenings MA₁ and MA₂ shown in FIG. 4), whether conscious or unconscious. In some implementations, the wake-after-sleep onset (WASO) is defined as a persistent wake-after-sleep onset (PWASO) that only includes the total durations of awakenings having a predetermined length (e.g., greater than 10 seconds, greater than 30 seconds, greater than 60 seconds, greater than about 5 minutes, greater than about 10 minutes, etc.)

The sleep efficiency (SE) is determined as a ratio of the total time in bed (TIB) and the total sleep time (TST). For example, if the total time in bed is 8 hours and the total sleep time is 7.5 hours, the sleep efficiency for that sleep session is 93.75%. The sleep efficiency is indicative of the sleep hygiene of the user. For example, if the user enters the bed and spends time engaged in other activities (e.g., watching TV) before sleep, the sleep efficiency will be reduced (e.g., the user is penalized). In some implementations, the sleep efficiency (SE) can be calculated based at least in part on the total time in bed (TIB) and the total time that the user is attempting to sleep. In such implementations, the total time that the user is attempting to sleep is defined as the duration between the go-to-sleep (GTS) time and the rising time described herein. For example, if the total sleep time is 8 hours (e.g., between 11 PM and 7 AM), the go-to-sleep time is 10:45 PM, and the rising time is 7:15 AM, in such implementations, the sleep efficiency parameter is calculated as about 94%.

The fragmentation index is determined based at least in part on the number of awakenings during the sleep session. For example, if the user had two micro-awakenings (e.g., micro-awakening MA₁ and micro-awakening MA₂ shown in FIG. 4), the fragmentation index can be expressed as 2. In some implementations, the fragmentation index is scaled between a predetermined range of integers (e.g., between 0 and 10).

The sleep blocks are associated with a transition between any stage of sleep (e.g., the first non-REM stage, the second non-REM stage, the third non-REM stage, and/or the REM) and the wakefulness stage. The sleep blocks can be calculated at a resolution of, for example, 30 seconds.

In some implementations, the systems and methods described herein can include generating or analyzing a hypnogram including a sleep-wake signal to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof based at least in part on the sleep-wake signal of a hypnogram.

In other implementations, one or more of the sensors 130 can be used to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof, which in turn define the sleep session. For example, the enter bed time t_{bed} can be determined based at least in part on, for example, data generated by the motion sensor 138, the microphone 140, the camera 150, or any combination thereof. The go-to-sleep time can be determined based at least in part on, for example, data from the motion sensor 138 (e.g., data indicative of no movement by the user), data from the camera 150 (e.g., data indicative of no movement by the user and/or that the user has turned off the lights), data from the microphone 140 (e.g., data indicative of the using turning off a TV), data from the user device 170 (e.g., data indicative of the user no longer using the user device 170), data from the pressure sensor 132 and/or the flow rate sensor 134 (e.g., data indicative of the user turning on the respiratory therapy device 122, data indicative of the user donning the user interface 124, etc.), or any combination thereof.

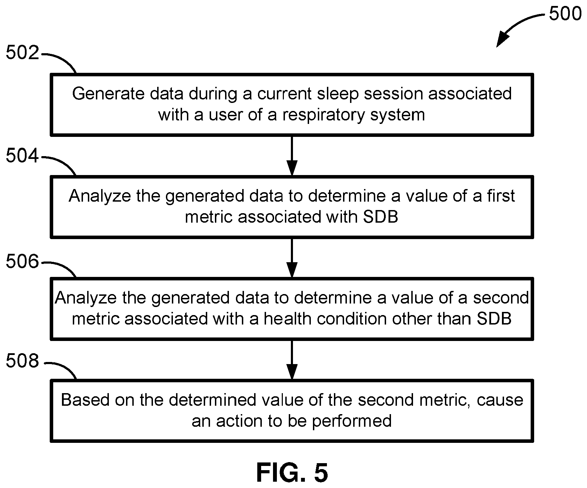

Referring to FIG. 5, a method 500 of monitoring a user during a sleep session as the user uses a respiratory therapy system (such as respiratory therapy system 12) is illustrated. Generally, a control system (such as control system 110 of system 100) is configured to carry out the various steps of method 600. A memory device (such as memory device 114 of system 100) can be used to store any type of data utilized in the steps of method 500 (or other methods). Often, users with SDB may have additional health conditions (also known as comorbidities) accompanying their SDB. Use of a respiratory therapy system (for example as a CPAP system) may generate data that can be analyzed to identify and/or monitor these other health conditions. The use of the respiratory therapy system can also in some cases treat the additional health conditions, as well as the SDB. By analyzing data generated during use of the respiratory therapy system, as well as additional data in some cases, various metrics related to the additional health conditions can be determined, and more effective treatment options for the additional health conditions can be determined.

Step 502 of method 500 includes generating data during a current sleep session that is associated with a user of a respiratory therapy system. The data can be generated by any suitable source, including a number of different sensors (such as sensor(s) 130). The sensors can be located within a housing of a respiratory therapy device (such as respiratory therapy device 122) used with the respiratory therapy system, or outside of the housing of the respiratory therapy device. The data can also be generated by a number of different devices, such as a smart watch, an activity tracker, a mobile phone, any number of external medical measurement devices, etc. The respiratory therapy system can include a respiratory therapy device that supplies pressurized air to the user's airway via, for example, a conduit and a user interface. The generated data can include data that is indicative of the pressure of the pressurized air, the flow rate of the pressurized air, and other properties of the pressurized air. The user interface can include a full facial mask that covers the user's mouth and nose, a nasal mask, or other types of user interfaces.

Step 504 of method 500 includes analyzing the generated data to determine the value of a first metric associated with sleep-disordered breathing (SDB). Step 506 of method 500 includes analyzing the generated data to determine the value of a second metric associated with a health condition of the user other than SDB. The first metric can include metrics that are used to analyze the user's sleep session. For example, if the respiratory therapy system is being used as a CPAP system, the first metric can be associated with using the CPAP system to minimize events, such as apnea events.

The first metric (and in some implementations the second metric) can include a respiration signal, a respiration rate, a respiration pattern, an inspiration amplitude, an expiration amplitude, an inspiration-expiration ratio, an occurrence of one or more events, a number of events per hour, a pattern of events, an average duration of events, a range of event durations, a ratio between the number of different events, a sleep stage, a time spent in each of a plurality of sleep stages, a pattern of sleep stages, an apnea-hypopnea index (AHI), a sleep score, a therapy score, a total sleep time, a total time in bed, a wake-up time, a rising time, a hypnogram, a total light sleep time, a total deep sleep time, a total REM sleep time, a total sleep time on therapy, a total sleep time off therapy a number of awakenings, a sleep-onset latency, a stress level, or any combination thereof.

In some implementations, the first metric is associated only with SDB and the second metric is associated only with the health condition. In some implementations, the first metric is associated with SDB and the health condition, and the second metric is associated only with the health condition. In some implementations, the first metric is associated only with SDB, and the second metric is associated with the health condition and SDB. In some implementations, the first metric is associated with SDB and the health condition. In some implementations, the second metric is associated with the health condition and SDB.

In some implementations, the second metric is associated with the presence of a cardiac condition. For example, the second metric can be associated with heart failure, a heart murmur, an increased heart rate, a heart attack, atrial fibrillation, heart arrhythmia, myocarditis, cardiac autonomic neuropathy, chronic inflammatory conditions such as atherosclerosis (the repetitive drops in nocturnal blood oxygen levels caused by SDB can result in intermittent hypoxia, which has been shown to be associated with systemic inflammation), and others. The second metric can include a heart rate variability (a lack of a decrease in heart rate variability during a sleep session can indicate that the heart is stressed and not reacting correctly to being asleep); a standard deviation in beat-to-beat intervals (also known as SDNN, and can be related to the heart's recovery capability); root mean square of successive differences between adjacent beats (also known as RMSSD, and can be related to the heart's recovery capability); a cardiac output (a decreased cardiac output (e.g., smaller amount of blood pumped per time interval) can be indicative of hypertension and/or heart failure); a structure of the atrial chambers (a modified structure can be indicative of a risk of atrial fibrillation); a tidal volume (instability in tidal volume can be indicative of heart failure); a duration of apnea, a duration of expiration, a gradient of expiration, an envelope of the flow signal (which can show the level of stability in the user's breathing), a respiration rate; and others.

In some implementations, the second metric is associated with the user's clinical stress level, or sympathetic activity. For example, the second metric can include a heart rate, a heart rate variability, a skin conductance (e.g., a galvanic skin response), an arterial pulse speed, an arterial pulse shape, an arterial pulse volume, an arterial pulse amplitude, or any combination thereof. Further, one or more of these various measurements can be used to quantify the stress level of the user, so that the second metric itself includes a quantifiable measure of the user's stress level. The second metric can also be a measure of the user's stress response to various different types of apnea events. For example, the second metric for one user can indicate that the user experiences elevated stress levels (which could be measured using heart rate, heart rate variability, skin conductance, arterial pulse characteristics, etc.) only in response to longer apnea events and/or apnea events with a significant reduction in inspiration volume. However, the second metric for a different user can indicate that that user experiences elevated stress levels in response to shorter apnea events and/or apnea events with a minor reduction in inspiration volume, as well as apnea events having longer durations and/or a significant reduction in inspiration volume. The second metric could also indicate how much the stress levels of that user increase as the severity of the apnea event increases. Thus, the second metric in some implementations can include a link between the user's stress response and various different types of apnea events, a percentage of different types of apnea events that result in different stress responses, and others.

In further implementations, the respiratory therapy system may be configured to learn an association between the second metric and the respiratory therapy system therapy (e.g., CPAP pressure). In yet further implementations, a respiratory therapy level (e.g., CPAP pressure level) may be varied to augment learning the association between therapy and the second metric, and in further implementations still, a therapy level may be adjusted to control the second metric (e.g., CPAP pressure may be increased or decreased to reduce a metric associated with stress or sympathetic activity).

In some implementations, the second metric is associated with the user's breathing during the sleep session. For example, the second metric can include the cadence of the user's breathing (e.g., how quickly or slowly the user breathes, and/or if there are any starts and stops in the user's breathing), an amplitude of the breathing of the user (e.g., to the volume of air that the user breathes in and/or breathes out), a time constant of expiration (e.g., the time constant of the exponential decay of the lung volume during expiration), a shape of expiration (e.g., a shape of a plot of the lung volume over time during expiration), a magnitude of expiration (e.g., a volume or force of expiration), a speed of expiration (e.g., how quickly the lung volume decreases during expiration), a time constant of inspiration (e.g., the time constant of the exponential increase of the lung volume during inspiration), a shape of inspiration (e.g., a shape of a plot of the lung volume over time during inspiration), a magnitude of inspiration (e.g., a volume and/or force of inspiration), a speed of inspiration (e.g., how quickly the lung volume increases during inspiration), a tidal volume, a rate of the breathing, or other breathing metrics (e.g., respiratory-associated metrics).

These breathing metrics can be associated with any number of different health conditions. For example, the cadence of the user's breathing and/or the tidal volume can be associated with obesity, COPD, pneumonia, asthma, and other conditions. A user being short of breath (e.g., the cadence of the user's breathing is rapid and/or the tidal volume is shallow) can indicate that the user is obese, or has COPD, pneumonia, asthma, etc. In another example, the shape of inspiration and/or expiration can be indicative of the presence of an obstruction in the user's airway. In a further example, the tidal volume can be associated with heart failure. Instability in the tidal volume (e.g., different volumes for different breaths) can be indicative of the presence of heart failure, or the risk of developing failure. In a further example, any one or more of (i) the cadence of breathing, (ii) the shape, speed, and magnitude of inspiration, and (iii) the shape, speed, and magnitude of expiration can be indicative of the presence of Cheyne-Stokes respiration.

In another example, the second metric can be associated with when the user is awake or asleep during the sleep session, and can include the amount of time it took to fall asleep, the total amount of time spent awake, the total amount of time spent asleep, the time that the user fell asleep, the time that the user woke up, a consistency in the user's breathing rate, and others. The metrics can indicate that the user was awake for a longer time than desired, was asleep for a shorter time than desired, that the user's breathing did not slow down and become consistent (indicating that the user is asleep) until later in the sleep session than desired, etc., which can all indicate the user suffers from insomnia. Information related to the sleep staging, such as a hypnogram (e.g., hypnogram 400 of FIG. 3) can also be used to aid in determining whether the user has insomnia.

These metrics or other metrics can also be indicative of the presence of other neurological conditions, such as anxiety, claustrophobia, stress, and others. In general, the second metric can be associated with cardiac conditions, respiratory conditions, neurological conditions, and other types of conditions. In general, the metrics associated with neurological conditions can include heart rate (high heart rate can be indicative of anxiety); blood pressure (high blood pressure can be indicative of anxiety); sleep onset latency (low sleep onset latency can be indicative of anxiety); an arousal threshold (e.g., how easily the user wakes up - the arousal threshold being low can be indicative of anxiety); an amount of physical movement during sleep session (e.g., uncontrolled leg movement can be indicative of restless leg syndrome); metrics related to awakenings during the sleep session (erratic sleep-wake patterns can be indicative of disruption of circadian rhythms and can lead to morning depression, and can also be indicative of nocturnal epilepsy); interface leak rate (a high rate of leaks from the user's interface, such as user interface 124, can indicate that the user interface is being worn looser as a result of claustrophobia); a number of times the user interface is removed during the sleep session (a large amount can be indicative of claustrophobia); a various metrics related to the user's answers to a questionnaire, as discussed in more detail below.

Step 508 of method 500 includes causing some action to be performed, based at least in part on the determined value of the second metric. In some implementations, the second metric is indicative of the identity, presence, and/or severity of the health condition in the user, and thus can be used to determine the identity, presence, and/or severity of the health condition in the user. A number of different actions can then be taken.

In some implementations, based at least in part on the determined value of the second metrics, one or more settings of the respiratory therapy system can be adjusted. In one example, the respiratory therapy system is initially operated as a positive airway pressure system, such as a CPAP system. In response to the value of the second metric indicating the presence of a cardiac condition (such as heart failure) or an increased risk of developing a cardiac condition (such as heart failure), one or more settings of the respiratory therapy device can be adjusted (manually or automatically by the control system) so that the respiratory therapy system operates as an adaptive servo-ventilation system. Cardiac conditions (such as heart failure) often result in apneas that are not treatable with a CPAP system, and the use of a CPAP system can often worsen the occurrence and/or severity of these apneas. When the respiratory therapy system operates as an adaptive servo-ventilation system, the respiratory therapy system can monitor the user's breathing, and add volume to the user's breaths to ensure tidal volume stability, and reduce the occurrence and/or severity of these apneas.

In another example, settings related to the supply of the pressurized air can be modified, such as the pressure of the air, the flow rate of the air, the ramp time of the pressurized air (e.g., how long it takes for the pressure of the air to increase to the desired pressure (e.g., the desired therapy pressure) from the beginning of the use of the respiratory therapy system), a humidity of the pressurized air, and others. A medicament or other substance can also be supplied and/or injected into user's airway via the pressurized air. The medicament can be configured to aid the user in falling asleep. The substance could be a scent configured to calm the user and aid the user in falling asleep. In one example, the volume of the respiratory therapy system when operating at high pressure can impact a user's insomnia, and in some cases worsen the user's insomnia. If the second metric indicates that the user has insomnia, the ramp time can be increased, which gives the user a longer time to fall asleep until the respiratory therapy system achieves the desired pressure. In a further example, if the second metric indicates the presence of insomnia, the therapy response sensitivity ramp time can be increased, and/or the air pressure can be maintained at a relatively low level (e.g., less than the desired therapy, which can increase faster in the presence of milder events such as snore or flow limitation), until the respiratory therapy system detects that the user has fallen asleep (e.g., based at least in part on the sleep-wake signal).

In another example, the humidity of the pressurized air can be increased for users suffering from COPD, asthma, allergies, a sinus infection, rhinitis, or other respiratory-related conditions. In a further example, if the second metric indicates that the user is suffering from a condition that can be treated by inhaled medication, a medicament can be supplied and/or injected into the user's airway. In some of these implementations, the value of the first metric related to SDB may impact how the settings of the respiratory therapy system are adjusted. In still other implementations, adjusting the settings can include changing the type of user interface currently being used. The microphone 140 and/or the speaker 142 can be used to characterize the user interface, for example by directing an acoustic signal toward the user interface, and then analyzing acoustic data associated with a reflection of the acoustic signal. Once the type of user interface currently being used is determined, a recommendation can be generated to change the type of user interface, based at least in part on the determined value of the second metric. In general, the settings of the respiratory therapy system have an intended therapy effect. The settings of the respiratory therapy system can be adjusted as needed to modify the intended therapy effect.

In a further example, adjusting the settings of the respiratory therapy system can include adjusting the pressure of the pressurized air to account for the CO₂ level around the interface. If the user's interface is leaking and/or if the user's ventilation rate is low, the pressure of the pressurized air can be decreased.

In some implementations, a notification and/or report can be generated and transmitted to the user and/or a third party. The third party could include a friend, family member, spouse, significant other, caretaker, healthcare provider, etc. The notification and/or report can identify the condition that was detected and/or the severity of the detected condition. The notification and/or report can also recommend future therapy to help treat the condition. For example, of the second metric indicates the presence of insomnia, the notification and/or report can suggest cognitive behavioral therapy. The notification and/or report can be displayed to the user on an electronic display device, such as display device 172 of user device 170.

In some implementations, the techniques of method 500 can be used to monitor the efficacy of the use of the respiratory therapy system in treating the health condition. In these implementations, the second metric is tracked over multiple sleep sessions, and the value of the second metric is determined for each sleep session. The settings of the respiratory therapy system for each sleep session can be determined, along with the duration of use of the respiratory therapy system for each sleep session. The settings and duration of use of the respiratory therapy system can be correlated with any change in the presence and/or severity of the health condition. The optimal settings and/or duration of use to treat the health condition can then be determined.

In some implementations, the second metric is related to the stress level of the user, as discussed above. In these implementations, the action can include adjusting various settings of the respiratory therapy system, based on the determined stress level of the user. In one example, the stress level of the user is used to adjust in real-time how the respiratory therapy system responds to an apnea event. The respiratory therapy system may be operated as a positive airway pressure system that supplies pressurized air to the user's airway at a constant pressure (a CPAP system) or at two different constant pressures for the user's inspiration and exhalation (a BiPAP system or a VPAP system). If the user is experiencing elevated stress levels, the respiratory therapy system can increase the pressure of the supplied air when a severe apnea event is detected. Increasing the pressure of the air can aid in ending severe apnea events more quickly, and/or minimizing the inspiration reduction that occurs during the severe apnea event. Increasing the pressure can have negative side effects, such as drying out the user's airway, and potentially waking the user up, and thus is generally not done. However, if the user experiences elevated stress levels during the sleep session (and particularly during the severe apnea event), the benefit of ending the severe apnea event more quickly by increasing the pressure of the air can outweigh any drawbacks from the increased pressure. Thus, the respiratory therapy system in these implementations can increase the pressure of the air in response to a severe apnea event only if the user's stress levels are elevated. If the user's stress levels are not elevated, the respiratory therapy system can maintain the same pressure level, even when a severe apnea event occurs.

In some implementations, the pressure is increased in response to a severe apnea event if the user is experiencing elevated stress levels during the sleep session generally. Once the severe apnea event ends, the pressure can be decreased back to its standard level. In other implementations, the pressure is increased in response to a severe apnea event if the user experiences elevated stress levels during the severe apnea event. Once the severe apnea event ends and/or the user's stress levels return to normal, the pressure can be decreased back to its standard level. In further implementations, this increase in pressure could occur during mild apnea events, not just severe apnea events. Thus, in some implementations, the respiratory therapy system can increase the pressure of the air delivered to the user's airway in response to an apnea event (severe or mild), but only if the second metric indicates that the user's stress levels are increased. If the second metric does not indicate that the user's stress levels are elevated during the apnea event, the respiratory therapy system can continue operating at its standard pressure(s).

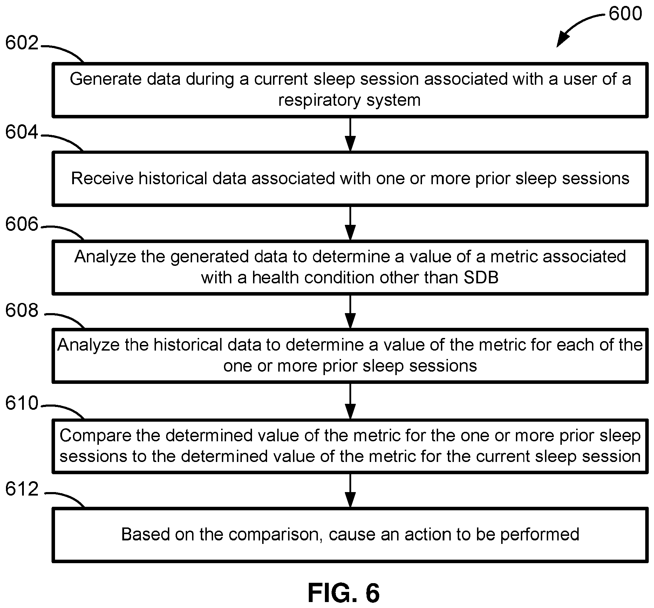

Referring to FIG. 6, a method 600 of monitoring a user during a sleep session as the user uses a respiratory therapy system (such as respiratory therapy system 12) is illustrated. Generally, a control system (such as control system 110 of system 100) is configured to carry out the various steps of method 600. A memory device (such as memory device 114 of system 100) can be used to store any type of data utilized in the steps of method 600 (or other methods).

Step 602 of method 600 includes generating data during a current sleep session associated with a user of a respiratory therapy system. Step 602 of method 600 is the same as or similar to method 502 of method 500. Step 604 includes receiving historical data associated with one or more prior sleep sessions. Generally, the historical data can be the same type as the data generated during the current sleep session, except that the historical data is associated with one or more prior sleep sessions of the user.

Step 606 of method 600 includes analyzing the generated data to determine the value of a metric associated with a health condition of the user other than SDB. Step 606 of method 600 is the same as or similar to step 506 of method 500. Thus, the metric for which the value is determined can be the second metric for which the value is determined in step 506. Step 608 of method 600 includes analyzing the historical data to determine a value of the metric for each of the one or more prior sleep sessions. Step 610 of method 600 includes comparing the determined value of the metric for the one or more prior sleep sessions to the determined value of the metric for the current sleep session. The comparison can reveal whether the severity of the condition in the user increased or decreased over time.

Finally, step 612 of method 600 includes causing an action to be performed based at least in part on the comparison. If the comparison of step 610 indicates that the severity of the health condition has increased, a variety of different actions can be taken. In some implementations, a notification can be sent to the user or a third party (such as a friend, family member, spouse, partner, caretaker, or healthcare provider). The notification can provide information about the increased severity of the health condition. The notification can be displayed to the user on an electronic display device, such as user device 170. In another implementation, one or more settings of the respiratory therapy system can be modified to better treat the health condition.

In yet another implementation, a recommendation for future therapy can be transmitted to the user. The recommendation for future therapy can include a recommendation to visit a doctor or a dentist, a recommendation to undergo a medication regimen or a medical regimen, a recommendation to modify settings of the respiratory therapy system, a recommendation to use a separate device to treat the health condition (such as using a portable oxygen concentrator), or a recommendation to use a separate device to confirm the increased severity of the health condition.

The separate device can be any device that may be used to confirm the increased severity. For example, the device may be a medical measurement device that the user can use to take a measurement of some property or parameter that is indicative of the severity of the health condition. The separate device can include a pulse oximeter configured to measure an oxygen saturation of the user, a blood pressure monitor configured to monitor a blood pressure of the user, a heart rate monitor configured to measure a heart rate of the user, a blood glucose meter configured to measure a blood glucose level of the user, an electroencephalography (EEG) device configured to measure brain activity (to monitor central arousals and/or sleep stages during the sleep session), an electrooculography (EOG) device configured to detect eye movements during the sleep session, a wearable device to detect movements during the sleep session, a mattress sensor to detect movements during the sleep session, or other devices.

In still other implementations, the comparison between data from the current sleep session and the past sleep sessions can indicate that the severity of the health condition has increased, decrease, or has not changed, or has decreased. If the severity of the health condition has increased, the action can include recommending to the user that they adjust the settings and/or use of the respiratory therapy system to increase the intended therapy effect of the respiratory therapy system, commensurate with the increased severity of the health condition. If the severity of the health condition has decreased or stayed the same, the action can include recommending to the user that they either continue using the respiratory therapy system with the current settings, or to adjust settings and/or use of the respiratory therapy system to decrease the intended therapy effect of the respiratory therapy system commensurate with the unchanged or decreased severity of the health condition. Other types of actions can also be taken.

In additional implementations, the data from the prior sleep session can be analyzed to predict the future status of various health conditions of the use. This can include predicting a suitable time in the future for any type of intervention or therapy change, and sending a notification and/or report regarding predict future intervention or therapy change to the user or to a third party. The prediction could also be continually updated as more data is obtained from each sleep session.

In some implementations, the historical data from the one or more prior sleep sessions is analyzed to track the user's stress levels over time, and the second metric includes a measure of the user's stress levels during the current sleep session. If a comparison indicates that the user's stress levels during sleep sessions are not decreased, the settings of the respiratory therapy system can be adjusted to aid in reducing the user's stress levels. For example, the settings of the respiratory therapy system could be adjusted to increase the ramp-up time of the pressurized air delivered to the user's airway or to decrease the pressure of the pressurized air delivered to the user's airway. Generally, when the user initially begins to use the respiratory therapy system at the beginning of the sleep session, the respiratory therapy system ramps up the pressure of the air supplied to the user's airway from a low initial pressure to a higher working pressure over a first time period (which could be 5 minutes, 10 minutes, 30 minutes, 1 hour, etc.). The time it takes for the pressure to increase from the initial pressure to the working pressure can be referred to as the ramp-up time. If the user's stress levels are elevated (for example in response to initially donning the user interface), the action can include increasing the ramp-up time of the respiratory therapy system, such that the pressure of the air supplied to the user increases from the initial pressure to the working pressure over a second time period that is greater than the first time period. The action could additionally or alternatively include decreasing the working pressure of the respiratory therapy system. In these implementations, the working pressure could be modified to be a modified working pressure that is less than the original working pressure.

In some implementations, the historical data can be used to determine the user's baseline stress levels during different types of apnea events, and the second metric can include a measurement of the user's stress levels during the current sleep session. If the second metric indicates that the user is experiencing elevated stress levels (as compared to the baseline) when experiencing certain apnea events, the pressure of the pressurized air delivered to the user's airway can be increased to aid in reducing the duration and/or severity of the apnea event, even if an apnea event of that duration and/or severity would normally be treated with an increased pressure. Similarly, if the second metric indicates that the user is not experiencing elevated stress levels when experiencing certain apnea events, the pressure of the pressurized air delivered to the user's airway can be kept constant, even if an apnea event of that duration and/or severity would normally be treated with an increased pressure.

While method 600 generally refers to comparing data from the current sleep session to data from prior sleep sessions, method 600 is generally applicable to any comparison between different sets of data generated during different sleep sessions. Thus, data generated during a current sleep session can also be compared to data generated during subsequent sleep sessions, in order to determine the value of the second metric for the current and subsequent sleep sessions, and take some action based at least in part on the comparison.

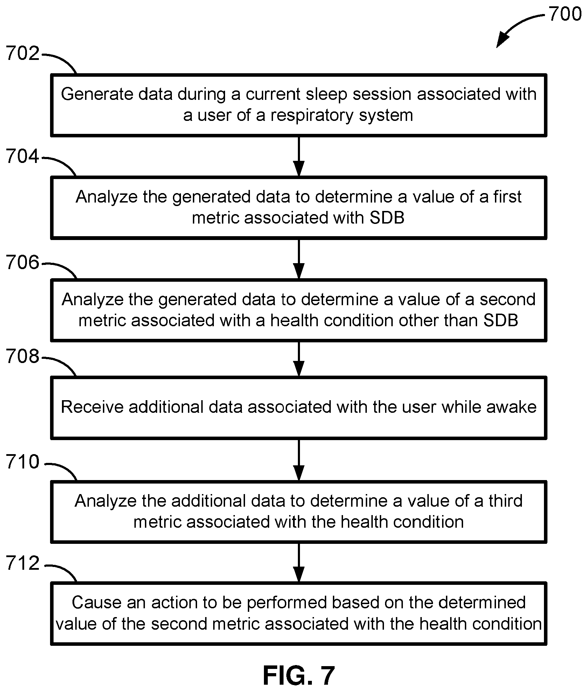

Referring to FIG. 7, a method 700 of monitoring a user during a sleep session as the user uses a respiratory therapy system (such as respiratory therapy system 12) is illustrated. Generally, a control system (such as control system 110 of system 100) is configured to carry out the various steps of method 700. A memory device (such as memory device 114 of system 100) can be used to store any type of data utilized in the steps of method 700 (or other methods).

Step 702 of method 700 includes generating data during a current sleep session that is associated with a user of the respiratory therapy system. Step 702 of method 700 is generally the same as or similar to step 602 of method 600 and/or step 502 of method 500. Step 704 of method 700 includes analyzing the generated data to determine the value of a first metric associated with sleep-disordered breathing (SDB). Step 704 of method 700 is generally the same as or similar to step 504 of method 500. Step 706 of method 700 includes analyzing the generated data to determine the value of a second metric that is associated with a health condition other than SDB. Step 706 of method 700 is generally the same as or similar to step 506 of method 500 and step 606 of method 600.

Step 708 of method 700 includes receiving additional data that is associated with the user while the user is awake. Generally, the additional data is associated with the user, but not associated with the user's use of the respiratory therapy system. This data can include demographic information such as an age of the user, a sex of the user, a gender of the user, a geographic location of the user, a height of the user, a weight of the user, a neck size of the user, and an occupation of the user; medical information associated with the user, such as a smoking status of the user; audio data associated with the user, such as answers of the user to a questionnaire or other data related to the user speaking; and other types of additional data.

The questionnaire can include questions that are designed to elicit further information from the user about themselves. The questions can be related to the quality and/or amount of the user's sleep, the user's health, the user recent activities, the user's physical activity/exercise, the user's stress levels, the user's physical health, the user's mental health, other data, or any combination thereof. The data associated with the user's answers to the questionnaire can include the content of the user's answers.

However, in some implementations, the data can also include how the user actually answers the questions. Properties including the tone of the user's voice, the cadence of the user's speech, the structure of the provided answers (e.g., a full sentence versus single words), the speed and accuracy of the user's answers, etc., can provide insight into different conditions, including neurological conditions such as anxiety, depression, etc. For example, a faster cadence, a monotone/limited tone, high ratio of breathing sounds to voice during the answer, a large separation between words, and/or any tremors in the user's voice can indicate the presence of neurological conditions such as anxiety and/or depression.

In some implementations, the questions can be displayed to the user via the display device 128 of the respiratory therapy system 120, and/or the display device 172 of the user device 170. In other implementations, the questions can be transmitted to the user (e.g., played out loud) via the speaker 142. The user's answers to the questionnaire can be manually inputted (for example, via the display device 128 of the respiratory therapy system 128, or the user device 170). However, the user can also speak their answers aloud, and a sensor (such as microphone 140) can generate audio data indicative of the user's answers. The audio data is representative of properties or features in the user's answers indicative of health conditions of the user.

The additional data can also include audio data other than just the user's answers to the questionnaire. For example, the user could be recorded during the day (for example using microphone 142 or other devices). Audio data associated with the user's speech and other sounds made by the user (e.g., coughing, wheezing, etc.) can be indicative of a variety of different conditions, including respiratory conditions (e.g., asthma, pneumonia, allergies, respiratory infection, etc.) and neurological conditions (depression, anxiety, bi-polar disorder, etc.).

Step 710 of method 700 includes analyzing the received additional data (the data associated with the user while awake) to determine a value of a third metric that is associated with the health condition. Step 712 of method 700 includes causing an action to be performed based at least in part on the determined value of the third metric.

Generally, the third metric associated with the health condition is different than the second metric associated with the health condition. By determining the value of the third metric from the additional data associated with the user while awake, a more accurate representation of the health condition can be obtained, and an action can be taken.

In some implementations, the value of the second metric can be adjusted based at least in part on the value of the third metric. In these implementations, the precision of the determined value of the second metric may be limited when based at least in part on only the data associated with use of the respiratory therapy system. However, the value of the second metric may be more precisely determined by taking into account the data associated with the user while awake, and/or the value of the third metric.

In other implementations, the value of the second metric may indicate that the user could have a plurality of different health conditions. However, it may be impossible to determine which of the plurality of health conditions the user is actually suffering from, based at least in part on only the value of the second metric. The value of the third metric may provide more details, that enables which of the plurality of health conditions the user is suffering from to be determined. In still other implementations, the reverse may be true. The value of the third metric may indicate the possibility the user is suffering from a plurality of different health conditions, and the value of the second metric can be then used to determine which of the plurality of health conditions the user is suffering from.

In still other implementations, only the identity of the health condition the user is suffering from can be determined from the second metric (or the third metric). The value of the third metric (or the second metric) can then be used to determine the severity of the health condition.

Generally, any of methods 500, 600, and 700 can be implemented using a system having a control system with one or more processors, and a memory device storing machine readable instructions. The controls system can be coupled to the memory device, and methods 500, 600, and 700 can be implemented when the machine readable instructions are executed by at least one of the processors of the control system. Methods 500, 600, and 700 can also be implemented using a computer program product (such as a non-transitory computer readable medium) comprising instructions that when executed by a computer, cause the computer to carry out the steps of methods 500, 600, and 700.

One or more elements or aspects or steps, or any portion(s) thereof, from one or more of any of claims 1-125 below can be combined with one or more elements or aspects or steps, or any portion(s) thereof, from one or more of any of the other claims 1-125 or combinations thereof, to form one or more additional implementations and/or claims of the present disclosure.

While the present disclosure has been described with reference to one or more particular embodiments or implementations, those skilled in the art will recognize that many changes may be made thereto without departing from the scope of the present invention. It is also contemplated that additional implementations according to aspects of the present disclosure may combine any number of features from any of the implementations described herein.

## Claims

1. A system (100) for monitoring a sleep session of a user (210), the system (100) comprising:
a respiratory therapy system (120) including:
a respiratory therapy device (122) configured to supply pressurized air, the respiratory therapy device (122) including a housing having one or more sensors (130) positioned therein; and
a user interface (124) coupled to the respiratory therapy device (122) via a conduit (126), the user interface (124) being configured to engage the user (210) and aid in directing the supplied pressurized air to the airway of the user (210);
a memory device (114) storing machine-readable instructions; and
a control system (110) coupled to the memory device (114), the control system (110) including one or more processors (112) configured to execute the machine-readable instructions to:
cause data associated with the user (210) to be generated (502), during a current sleep session, the data including at least a first portion that is generated by the one or more sensors (130) that are positioned in the housing of the respiratory therapy device (122);
analyze (504) the generated data to determine a value of a first metric that is associated with a sleep disordered breathing (SDB) condition;
analyze (506) the generated data to determine a value of a second metric that is associated with a health condition other than the SDB condition, the health condition including insomnia, wherein the second metric includes a total time in bed, a total sleep time, a total wake time, a sleep onset latency, a wake-after-sleep-onset parameter, a sleep efficiency, a fragmentation index, an amount of time to fall asleep, a consistency of breathing rate, a fall asleep time, a wake time, a rate of sleep disturbances, a number of movements, or any combination thereof;
**characterized in that** the one or more processors are further configured to execute the machine-readable instructions to:
in response to the determined value of the second metric indicating a presence of insomnia, cause (508) an action to be performed, the action including adjusting one or more settings of the respiratory therapy system, the adjusting including:
(i) decreasing a pressure of the pressurized air below a desired therapy pressure, and in response to determining that the user has fallen asleep, increasing the pressure of the pressurized air to at least the desired therapy pressure; and/or
(ii) increasing a ramp time of the pressurized air, where the ramp time is the time it takes for the pressure of the air to increase to the desired therapy pressure from the beginning of the use of the respiratory therapy system.

2. The system (100) of claim 1, wherein the value of the second metric is indicative of a presence of the health condition, a severity of the health condition, or both; and/or
wherein the one or more processors (112) of the control system (110) are further configured to execute the machine-readable instructions to determine an identity of the health condition based at least in part on the determined value of the second metric, determine a severity of the health condition based at least in part on the determined value of the second metric, or both; and/or
wherein the first metric is associated only with SDB, and the second metric is associated only with the health condition; and/or
wherein the first metric includes a respiration signal, a respiration rate, a respiration pattern, an inspiration amplitude, an expiration amplitude, an inspiration-expiration ratio, an occurrence of one or more events, a number of events per hour, a pattern of events, an average duration of events, a range of event durations, a ratio between the number of different events, a sleep stage, a time spent in each of a plurality of sleep stages, a pattern of sleep stages an apnea-hypopnea index (AHI), a sleep score, a therapy score, a total sleep time, a total time in bed, a wake-up time, a rising time, a hypnogram, a sleep disturbance index, a total light sleep time, a total deep sleep time, a total REM sleep time, a total sleep time on therapy, a total sleep time off therapy, a number of awakenings, a sleep-onset latency, or any combination thereof; and/or
wherein the one or more sensors (130) positioned within the housing of the respiratory therapy device (122) include a flow rate sensor (134), a pressure sensor (132), a microphone (140) or any combination thereof; and/or
wherein at least a second portion of the data is generated by one or more sensors (130) positioned external to the respiratory therapy device; and/or
wherein the second metric is associated with breathing of the user (210) during the current sleep session; and/or
wherein the second metric includes a cadence of the breathing, an amplitude of the breathing of the user (210), a time constant of expiration, a shape of expiration, a magnitude of expiration, a speed of expiration, a time constant of inspiration, a shape of inspiration, a magnitude of inspiration, a speed of inspiration, a tidal volume, a rate of the breathing, or any combination thereof.

3. The system (100) of claim 2, wherein the second metric includes the cadence of the breathing and the health condition is obesity, chronic obstructive pulmonary disorder (COPD), pneumonia, asthma, Cheyne-Stokes respiration, heart failure, or any combination thereof; optionally, wherein the cadence of the breathing indicates shortness of breath and is associated with a presence of obesity; and/or
wherein the second metric includes a shape of expiration, and wherein the health condition is a presence of on obstruction in an airway of the user (210); and/or
wherein the second metric includes a tidal volume, and wherein an instability in the tidal volume indicates a presence of heart failure or a risk of developing heart failure; optionally, wherein the respiratory therapy system (120) is initially operated as a positive airway pressure system, and wherein in response to the second metric indicating a presence of heart failure or a risk of developing heart failure, the action includes adjusting one or more settings of the respiratory therapy system (120) to operate as an adaptive servo-ventilation system.

4. The system (100) of any one of claims 1 to 3,
wherein in response to the second metric indicating the presence of insomnia, the action includes transmitting a recommendation for future therapy to the user (210) or to a third party;
optionally, wherein the future therapy includes cognitive behavioral therapy; and
the health condition includes a cardiac condition, and wherein the second metric includes a heart rate variability, a standard deviation in beat-to-beat intervals of a heart of the user (210), a root mean square of successive differences between adjacent beats of the heart of the user (210), a cardiac output, a structure of atrial chambers of the heart of the user (210), a tidal volume , a duration of apnea, a duration of expiration, a gradient of expiration, an envelope of the flow signal, a respiration rate, or any combination thereof; and
optionally, wherein the respiratory therapy system (120) is initially operated as a positive airway pressure system, and in response to the second metric indicating the presence of the cardiac condition, the action includes adjusting one or more settings of the respiratory therapy system (120) to operate as an adaptive servo-ventilation system; and/or
the cardiac condition includes heart failure, a heart murmur, an increased heart rate, a heart attack, atrial fibrillation, heart arrhythmia, myocarditis, cardiac autonomic neuropathy, chronic inflammation, or any combination thereof.

5. The system (100) of any one of claims 1 to 4, wherein:
the action includes transmitting a notification to the user (210) or to a third party, displaying a notification on an electronic display device (172), changing one or more settings of the respiratory therapy system, transmitting a recommendation for therapy to the user (210) or the third party, or any combination thereof; and/or
the generated data includes data indicative of a pressure of the pressurized air, a flow of the pressurized air, or both;
optionally, wherein the action includes modifying a flow rate of the pressurized air, supplying a medicament into the airway of the user (210) via the pressurized air, or both; and
optionally, wherein in response to the determined value of the second metric indicating a presence of insomnia, anxiety, claustrophobia, or any combination thereof, the action includes increasing the ramp time of the pressurized air; and/or
wherein in response to the determined value of the second metric indicating a presence of COPD, asthma, allergies, a sinus infection, rhinitis, or any combination thereof, the action includes increasing the humidity of the pressurized air, supplying the medicament into the airway of the user (210), or both.

6. The system (100) of any one of claims 1 to 5, wherein the one or more processors (112) of the control system (110) are further configured to execute the machine-readable instructions to:
track the second metric over a plurality of sleep sessions;
determine the value of the second metric for each of the plurality of sleep sessions;
determine (i) a duration of use of the respiratory therapy system (120) for each of the plurality of sleep sessions, (ii) values of one or more settings of the respiratory therapy system (120) for each of the plurality of sleep session, (iii) or both; and
identify (i) an optimal duration of use of the respiratory therapy system, (ii) optimal values of the one or more settings of the respiratory therapy system, or (iii) both, to treat the health condition; and/or
wherein the one or more processors (112) of the control system (110) are further configured to execute the machine-readable instructions to:
receive historical data associated with one or more prior sleep sessions of the user (210);
analyze the historical data to determine a value of the second metric for each of the one or more prior sleep sessions; and
compare the determined value of the second metric for each of the one or more prior sleep sessions to the determined value of the second metric for the current sleep session.

7. The system (100) of claim 6, wherein
the health condition includes a stress level of the user (210), and wherein in response to the comparison indicating that the stress level of the user (210) has increased, the action includes decreasing a pressure of pressurized air delivered to the user (210) by the respiratory therapy system; and/or
the one or more processors (112) of the control system (110) are configured to execute the machine-readable instructions to cause the action to be performed in response to the comparison indicating that a severity of the health condition has increased;
optionally, wherein the action includes transmitting a notification to the user (210) or to a third party, displaying a notification on an electronic display device (172), adjusting one or more settings of the respiratory therapy system, transmitting a recommendation for therapy to the user (210) or the third party, or any combination thereof;
optionally, wherein the recommendation for therapy includes a recommendation to visit a doctor, a recommendation to visit a dentist, a recommendation to use a separate device to perform a measurement indicative of a presence or a severity of the health condition, a recommendation to use a portable oxygen concentrator, or any combination thereof; and optionally
wherein the separate device is a pulse oximeter configured to measure an oxygen saturation of the user (210), a blood pressure monitor (180) configured to monitor a blood pressure of the user (210), a heart rate monitor configured to measure a heart rate of the user (210), a blood glucose meter configured to monitor a blood glucose level of the user (210), an electroencephalography device configured to monitor bran activity, an electrooculography device configured to monitor eye movement, a wearable device configured to measure physical movement of the user (210), a mattress sensor configured to measure physical movement of the user (210), or any combination thereof.

8. The system (100) of any one of claims 1 to 7, wherein the one or more processors (112) of the control system (110) are further configured to execute the machine-readable instructions to:
determine a severity of the health condition based at least in part on the determined value of the second metric for the current sleep session;
generate data during one or more subsequent sleep sessions;
determine a value of the second metric for each of the one or more subsequent sleep sessions;
determine an updated severity of the health condition based at least in part on the determined values of the second metric for each of the one or more subsequent sleep sessions; and
based at least in part on the updated severity of the health condition, cause the action to be performed;
optionally wherein:
the action includes transmitting a recommendation for future therapy to the user (210) based at least in part on the updated severity of the health condition; and/or
in response to the determined value of the second metric for each of the one or more subsequent sleep sessions indicating that the severity of the health condition has increased, the action includes adjusting one or more settings of the respiratory therapy system (120) to increase an intended therapy effect of the respiratory therapy system (120); and/or
in response to the determined value of the second metric for each of the one or more subsequent sleep sessions indicating that the severity of the health condition has increased, the action includes transmitting a recommendation for therapy to the user (210) or to a third party; and/or
wherein in response to the determined value of the second metric for each of the one or more subsequent sleep sessions indicating that the severity of the health condition has decreased, the action includes continuing to use the respiratory therapy system (120) with current settings, or adjusting one or more settings of the respiratory therapy system (120) to decrease an intended therapy effect of the respiratory therapy system.

9. The system (100) of any one of claims 1 to 8, wherein the one or more processors (112) of the control system (110) are further configured to execute the machine-readable instructions to:
receive additional data associated with the user (210) while awake;
analyze the additional data to determine a value of a third metric associated with the health condition; and
cause the action to be performed based at least in part on the determined value of the third metric associated with the health condition;
optionally, wherein the additional data includes an age of the user (210), a sex of the user (210), a gender of the user (210), a geographic location of the user (210), a height of the user (210), a weight of the user (210), a neck size of the user (210), medical information associated with the user (210), a smoking status of the user (210), an occupation of the user (210), answers of the user (210) to a questionnaire, how the user (210) provides the answers to the questionnaire, or any combination thereof.

10. The system (100) of claim 9, wherein the one or more processors (112) of the control system (110) are further configured to execute the machine-readable instructions to:
modify the determined value of the second metric based at least in part on the determined value of the third metric; and
cause the action to be performed based at least in part on the modified value of the second metric;
optionally, wherein:
the value of the second metric is associated with a plurality of health conditions, and wherein the one or more processors (112) of the control system (110) are further configured to execute the machine-readable instructions to determine an identity of the health condition of the user (210) from the plurality of health conditions based at least in part on the determined value of the third metric; and/or
the value of the third metric is associated with a plurality of health conditions, and wherein the one or more processors (112) of the control system (110) are further configured to execute the machine-readable instructions to determine an identity of the health condition of the user (210) from the plurality of health conditions based at least in part on the determined value of the second metric; and/or
the one or more processors (112) of the control system (110) are further configured to execute the machine-readable instructions to:
determine an identity of the health condition of the user (210) based at least in part on the determined value of the third metric; and
determine a severity of the health condition of the user (210) based at least in part on the determined value of the second metric; and/or
the one or more processors (112) of the control system (110) are further configured to execute the machine-readable instructions to:
determine an identity of the health condition of the user (210) based at least in part on the determined value of the third metric; and
determine a severity of the health condition of the user (210) based at least in part on the determined value of the second metric.

11. The system (100) of any one of claims 1 to 10, wherein the one or more processors (112) of the control system (110) are further configured to execute the machine-readable instructions to determine an identity of the health condition, a severity of the health condition, or both based at least in part on (i) the determined value of the second metric and (ii) additional data;
optionally, wherein the additional data includes demographic information associated with the user (210), medical information associated with the user (210), audio data associated with the user (210), or any combination thereof; and
optionally, wherein:
the demographic information includes as an age of the user (210), a sex of the user (210), a gender of the user (210), a geographic location of the user (210), a height of the user (210), a weight of the user (210), a neck size of the user (210), an occupation of the user (210), or any combination thereof; and/or
the medical information of the user (210) is obtained from a medical history of the user (210); and/or
the audio data associated with the user (210) includes answers of the user (210) to a questionnaire.

12. The system (100) of any one of claims 1 to 11, wherein the second metric is indicative of a stress level of the user (210), and includes a heart rate, a heart rate variability, a skin conductance, an arterial pulse speed, an arterial pulse shape, an arterial pulse volume, an arterial pulse amplitude, or any combination thereof; optionally, wherein in response to the second metric indicating an elevated stress level of the user (210) during an apnea event, the action includes increasing a pressure of the pressurized air.

13. The system (100) of any one of claims 1 to 12, wherein the determined value of the second metric indicates an elevated stress level of the user (210); optionally, wherein:
the action includes, (i) in response to the user (210) experiencing an apnea event, increasing a pressure of the pressurized air, (ii) in response to the apnea event ending, decreasing the pressure of the pressurized air, or (iii) both (i) and (ii); and/or
during operation of the respiratory therapy system, the respiratory therapy system (120) is configured to increase a pressure of the pressurized air from an initial pressure to a working pressure over a first time period;
optionally, wherein the action includes increasing a ramp-up time of the pressurized air supplied to the airway of the user (210), such that the pressure of the pressurized air increases from the initial pressure to the working pressure over a second time period that is greater than the first time period; and
optionally, wherein the action includes modifying the working pressure to be a modified working pressure that is less than the working pressure.

14. The system (100) of any one of claims 1 to 13, wherein the determined value of the second metric indicates that the user (210) is suffering from a respiratory-related condition, and wherein the action includes increasing a humidity of the pressurized air supplied to the airway of the user (210), optionally, wherein the respiratory-related condition includes chronic obstructive pulmonary disorder (COPD), asthma, allergies, a sinus infection, rhinitis, or any combination thereof.

## Patentansprüche

1. System (100) zum Überwachen einer Schlafsitzung eines Anwenders (210), wobei das System (100) umfasst:
ein Beatmungstherapiesystem (120), beinhaltend:
eine Beatmungstherapievorrichtung (122), die konfiguriert ist, Druckluft zuzuführen, wobei die Beatmungstherapievorrichtung (122) ein Gehäuse mit einem oder mehreren darin angeordneten Sensoren (130) beinhaltet; und
eine Anwenderoberfläche (124), die über eine Leitung (126) mit der Beatmungstherapievorrichtung (122) verbunden ist, wobei die Anwenderoberfläche (124) so konfiguriert ist, dass sie den Anwender (210) einbindet und dabei hilft, die zugeführte Druckluft in die Atemwege des Anwenders (210) zu lenken;
eine Speichervorrichtung (114), die maschinenlesbare Anweisungen speichert; und
ein Steuerungssystem (110), das mit der Speichervorrichtung (114) verbunden ist, wobei das Steuerungssystem (110) einen oder mehrere Prozessoren (112) beinhaltet, die konfiguriert sind, die maschinenlesbaren Anweisungen auszuführen, um:
zu verursachen, dass während einer aktuellen Schlafsitzung Daten, die mit dem Anwender (210) in Zusammenhang stehen, generiert werden (502), wobei die Daten zumindest einen ersten Teil beinhalten, der von einem oder den mehreren Sensoren (130) generiert wird, die im Gehäuse der Beatmungstherapievorrichtung (122) angeordnet sind;
die generierten Daten zu analysieren (504), um einen Wert einer ersten Messgröße zu bestimmen, die mit einer schlafbezogenen Atmungsstörung (SDB) in Zusammenhang steht;
die generierten Daten zu analysieren (506), um einen Wert einer zweiten Messgröße zu bestimmen, die mit einem anderen Gesundheitszustand als der SDB-Erkrankung in Zusammenhang steht, wobei der Gesundheitszustand Schlaflosigkeit beinhaltet, wobei die zweite Messgröße eine Gesamtzeit im Bett, eine Gesamtschlafzeit, eine Gesamtwachzeit, eine Einschlaflatenz, einen Parameter für das Aufwachen nach dem Einschlafen, eine Schlafeffizienz, einen Fragmentierungsindex, eine Einschlafzeit, eine Konsistenz der Atemfrequenz, eine Einschlafzeit, eine Aufwachzeit, eine Rate von Schlafstörungen, eine Anzahl von Bewegungen oder eine beliebige Kombination davon beinhaltet;
**dadurch gekennzeichnet, dass** der eine oder die mehreren Prozessoren ferner so konfiguriert sind, dass sie die maschinenlesbaren Anweisungen ausführen, um:
als Reaktion auf den bestimmten Wert der zweiten Messgröße, die auf das Vorliegen von Schlaflosigkeit hinweist, zu veranlassen (508), eine Maßnahme durchzuführen, wobei die Maßnahme die Anpassung einer oder mehrerer Einstellungen des Beatmungstherapiesystems beinhaltet, wobei die Anpassung Folgendes beinhaltet:
(i) Senken eines Drucks der Druckluft unter einen gewünschten Therapiedruck und, wenn festgestellt wird, dass der Anwender eingeschlafen ist, Erhöhen des Drucks der Druckluft zumindest auf den gewünschten Therapiedruck; und/oder
(ii) Erhöhen einer Anstiegszeit der Druckluft, wobei die Anstiegszeit die Zeit ist, die der Druck der Luft benötigt, um vom Beginn der Nutzung des Beatmungstherapiesystems an auf den gewünschten Therapiedruck anzusteigen.

2. System (100) nach Anspruch 1, wobei der Wert der zweiten Messgröße auf das Vorliegen des Gesundheitszustandes, einen Schweregrad des Gesundheitszustandes oder beides hinweist; und/oder
wobei der eine oder die mehreren Prozessoren (112) des Steuerungssystems (110) ferner so konfiguriert sind, dass sie die maschinenlesbaren Anweisungen ausführen, um eine Identität des Gesundheitszustands zumindest teilweise auf der Grundlage des bestimmten Wertes der zweiten Messgröße zu bestimmen, einen Schweregrad des Gesundheitszustands zumindest teilweise auf der Grundlage des bestimmten Wertes der zweiten Messgröße zu bestimmen oder beides; und/oder
wobei die erste Messgröße nur mit SDB und die zweite Messgröße nur mit dem Gesundheitszustand in Zusammenhang steht; und/oder
wobei die erste Messgröße ein Atmungssignal, eine Atmungsfrequenz, ein Atmungsmuster, eine Inspirationsamplitude, eine Expirationsamplitude, ein Inspirations-Expirations-Verhältnis, ein Auftreten eines oder mehrerer Ereignisse, eine Anzahl der Ereignisse pro Stunde, ein Ereignismuster, eine durchschnittliche Dauer der Ereignisse, einen Bereich der Ereignisdauern, ein Verhältnis zwischen der Anzahl verschiedener Ereignisse, ein Schlafstadium, eine in jedem von einer Vielzahl von Schlafstadien verbrachte Zeit, ein Schlafstadienmuster, einen Apnoe-Hypopnoe-Index (AHI), einen Schlaf-Score, einen Therapie-Score, eine Gesamtschlafzeit, eine Gesamtzeit im Bett, eine Aufwachzeit, eine Aufstehzeit, ein Hypnogramm, einen Schlafstörungsindex, eine Gesamtzeit im leichten Schlaf, eine Gesamtzeit im Tiefschlaf, eine Gesamtzeit im REM-Schlaf, eine Gesamtschlafzeit unter Therapie, eine Gesamtschlafzeit ohne Therapie, eine Anzahl der Aufwachvorgänge, eine Einschlaflatenz oder eine beliebige Kombination davon beinhaltet; und/oder
wobei der eine oder die mehreren im Gehäuse der Beatmungstherapievorrichtung (122) angeordneten Sensoren (130) einen Durchflusssensor (134), einen Drucksensor (132), ein Mikrofon (140) oder eine beliebige Kombination davon beinhalten; und/oder
wobei zumindest ein zweiter Teil der Daten von einem oder mehreren Sensoren (130) generiert wird, die außerhalb der Beatmungstherapievorrichtung angeordnet sind; und/oder
wobei die zweite Messgröße mit der Atmung des Anwenders (210) während der aktuellen Schlafsitzung in Zusammenhang steht; und/oder
wobei die zweite Messgröße eine Kadenz der Atmung, eine Amplitude der Atmung des Anwenders (210), eine Zeitkonstante der Exspiration, eine Form der Exspiration, eine Stärke der Exspiration, eine Geschwindigkeit der Exspiration, eine Zeitkonstante der Inspiration, eine Form der Inspiration, eine Stärke der Inspiration, eine Geschwindigkeit der Inspiration, ein Atemzugvolumen, eine Atemfrequenz oder eine beliebige Kombination davon beinhaltet.

3. System (100) nach Anspruch 2, wobei die zweite Messgröße die Atemfrequenz beinhaltet und der Gesundheitszustand Fettleibigkeit, chronisch obstruktive Lungenerkrankung (COPD), Lungenentzündung, Asthma, Cheyne-Stokes-Atmung, Herzinsuffizienz oder eine Kombination davon ist; wobei optional die Atemfrequenz auf Atemnot hinweist und mit dem Vorliegen von Fettleibigkeit in Zusammenhang steht; und/oder
wobei die zweite Messgröße eine Form der Ausatmung beinhaltet und wobei der Gesundheitszustand ein Vorhandensein einer Obstruktion in Atemwegen des Anwenders (210) ist; und/oder
wobei die zweite Messgröße ein Atemzugvolumen beinhaltet und wobei eine Instabilität des Atemzugvolumens auf ein Vorliegen einer Herzinsuffizienz oder das Risiko der Entwicklung einer Herzinsuffizienz hinweist; wobei optional das Beatmungstherapiesystem (120) anfänglich als System mit positivem Atemwegsdruck betrieben wird und wobei die Maßnahme als Reaktion auf die zweite Messgröße, die auf ein Vorliegen einer Herzinsuffizienz oder ein Risiko der Entwicklung einer Herzinsuffizienz hinweist, Anpassen einer oder mehrerer Einstellungen des Beatmungstherapiesystems (120) beinhaltet, um als adaptives Servoventilationssystem zu arbeiten.

4. System (100) nach einem der Ansprüche 1 bis 3,
wobei die Maßnahme als Reaktion auf den zweiten Messwert, der das Vorliegen von Schlaflosigkeit anzeigt, Übermitteln einer Empfehlung für eine künftige Therapie an den Anwender (210) oder an einen Dritten beinhaltet;
wobei optional die zukünftige Therapie eine kognitive Verhaltenstherapie beinhaltet; und
der Gesundheitszustand eine Herzerkrankung beinhaltet und wobei die zweite Messgröße eine Herzfrequenzvariabilität, eine Standardabweichung von Schlag-zuSchlag-Intervallen des Herzens des Anwenders (210), einen quadratischen Mittelwert aufeinanderfolgender Differenzen zwischen benachbarten Herzschlägen des Anwenders (210), ein Herzzeitvolumen, eine Struktur der Vorhofkammern des Herzens des Anwenders (210), ein Atemzugvolumen, eine Apnoedauer, eine Exspirationsdauer, einen Exspirationsgradienten, eine Hüllkurve des Flusssignals, eine Atemfrequenz oder eine beliebige Kombination davon beinhaltet; und
wobei optional das Beatmungstherapiesystem (120) zunächst als System mit positivem Atemwegsdruck betrieben wird und die Maßnahme in Reaktion auf die zweite Messgröße, die das Vorliegen der Herzerkrankung anzeigt, Anpassen einer oder mehrerer Einstellungen des Beatmungstherapiesystems (120) beinhaltet, um als adaptives ServoVentilationssystem zu arbeiten; und/oder
die Herzerkrankung Herzinsuffizienz, Herzgeräusche, eine erhöhte Herzfrequenz, einen Herzinfarkt, Vorhofflimmern, Herzrhythmusstörungen, Myokarditis, kardiale autonome Neuropathie, chronische Entzündungen oder eine Kombination davon beinhaltet.

5. System (100) nach einem der Ansprüche 1 bis 4, wobei:
die Maßnahme Übermitteln einer Benachrichtigung an den Anwender (210) oder an einen Dritten, Anzeigen einer Benachrichtigung auf einer elektronischen Anzeigevorrichtung (172), Ändern einer oder mehrerer Einstellungen des Beatmungstherapiesystems, Übermitteln einer Therapieempfehlung an den Anwender (210) oder an den Dritten oder eine beliebige Kombination davon beinhaltet; und/oder
die generierten Daten Daten beinhalten, die auf einen Druck der Druckluft, einen Durchfluss der Druckluft oder beides hinweisen;
wobei optional die Maßnahme Ändern einer Durchflussrate der Druckluft, Zuführen eines Medikaments in die Atemwege des Anwenders (210) über die Druckluft oder beides beinhaltet; und
wobei optional die Maßnahme in Reaktion auf den bestimmten Wert der zweiten Messgröße, die auf ein Vorliegen von Schlaflosigkeit, Angstzuständen, Klaustrophobie oder eine Kombination davon hinweist, Erhöhen der Anstiegszeit der Druckluft beinhaltet; und/oder
wobei die Maßnahme in Reaktion auf den bestimmten Wert der zweiten Messgröße, die auf ein Vorliegen von COPD, Asthma, Allergien, einer Nasennebenhöhlenentzündung, Rhinitis oder einer Kombination davon hinweist, Erhöhen der Luftfeuchtigkeit der Druckluft, Zuführen des Medikaments in die Atemwege des Anwenders (210) oder beides beinhaltet.

6. System (100) nach einem der Ansprüche 1 bis 5, wobei der eine oder die mehreren Prozessoren (112) des Steuerungssystems (110) ferner so konfiguriert sind, dass sie die maschinenlesbaren Anweisungen ausführen, um:
die zweite Messgröße über eine Vielzahl von Schlafphasen hinweg zu verfolgen;
den Wert der zweiten Messgröße für jede der Vielzahl vonSchlafsitzungen zu bestimmen;
(i) eine Nutzungsdauer des Beatmungstherapiesystems (120) für jede der Vielzahl von Schlafsitzungen, (ii) die Werte einer oder mehrerer Einstellungen des Beatmungstherapiesystems (120) für jede der Vielzahl von Schlafsitzungen, (iii) oder beides zu bestimmen; und
(i) eine optimale Nutzungsdauer des Beatmungstherapiesystems, (ii) optimale Werte der einen oder mehrerer Einstellungen des Beatmungstherapiesystems oder (iii) beides zur Behandlung des Gesundheitszustandes zu identifizieren; und/oder
wobei der eine oder die mehreren Prozessoren (112) des Steuerungssystems (110) ferner so konfiguriert sind, dass sie die maschinenlesbaren Anweisungen ausführen, um:
historische Daten zu empfangen, die mit einer oder mehreren früheren Schlafsitzungen des Anwenders (210) in Zusammenhang stehen;
die historischen Daten zu analysieren, um für jede der einen oder mehreren vorangegangenen Schlafphasen einen Wert für die zweite Messgröße zu bestimmen; und
den bestimmten Wert der zweiten Messgröße für jede der einen oder mehreren vorherigen Schlafsitzungen mit dem bestimmten Wert der zweiten Messgröße für die aktuelle Schlafsitzung zu vergleichen.

7. System (100) nach Anspruch 6, wobei:
der Gesundheitszustand einen Stresspegel des Anwenders (210) beinhaltet und wobei als Reaktion auf den Vergleich, der auf einen Anstieg des Stresspegels des Anwenders (210) hinweist, die Maßnahme Senken eines Drucks von Druckluft beinhaltet, die dem Anwender (210) vom Beatmungstherapiesystem zugeführt wird; und/oder
der eine oder die mehreren Prozessoren (112) des Steuerungssystems (110) so konfiguriert sind, dass sie die maschinenlesbaren Anweisungen ausführen, um zu veranlassen, dass die Maßnahme als Reaktion auf den Vergleich, der anzeigt, dass sich ein Schweregrad des Gesundheitszustands verschlechtert hat, durchgeführt wird;
wobei optional die Maßnahme Übermitteln einer Benachrichtigung an den Anwender (210) oder an einen Dritten, Anzeigen einer Benachrichtigung auf einer elektronischen Anzeigevorrichtung (172), Anpassen einer oder mehrerer Einstellungen des Beatmungstherapiesystems, Übermitteln einer Therapieempfehlung an den Anwender (210) oder an den Dritten oder eine beliebige Kombination davon beinhaltet;
wobei optional die Therapieempfehlung eine Empfehlung zum Besuch eines Arztes, eine Empfehlung zum Besuch eines Zahnarztes, eine Empfehlung zum Verwenden einer separaten Vorrichtung zum Durchführen einer Messung, die auf ein Vorhandensein oder einen Schweregrad des Gesundheitszustands hinweist, eine Empfehlung zur Verwendung eines tragbaren Sauerstoffkonzentrators oder eine Kombination davon beinhaltet; und optional
wobei es sich bei der separaten Vorrichtung um ein Pulsoximeter, das zum Messen einer Sauerstoffsättigung des Anwenders (210) konfiguriert ist, ein Blutdruckmessgerät (180), das zum Überwachen eines Blutdrucks des Anwenders (210) konfiguriert ist, ein Herzfrequenzmessgerät, das zum Messen einer Herzfrequenz des Anwenders (210) konfiguriert ist, ein Blutzuckermessgerät, das zum Überwachen eines Blutzuckerspiegels des Anwenders (210) konfiguriert ist, eine Elektroenzephalographievorrichtung, die zum Überwachen der Hirnaktivität konfiguriert ist, eine Elektrookulographievorrichtung, die zum Überwachen der Augenbewegungen konfiguriert ist, eine tragbare Vorrichtung, die zum Messen von körperlicher Bewegung des Anwenders (210) konfiguriert ist, einen Matratzensensor, der zum Messen körperlicher Bewegung des Anwenders (210) konfiguriert ist, oder eine beliebige Kombination davon handelt.

8. System (100) nach einem der Ansprüche 1 bis 7, wobei der eine oder die mehreren Prozessoren (112) des Steuerungssystems (110) ferner so konfiguriert sind, dass sie die maschinenlesbaren Anweisungen ausführen, um:
einen Schweregrad des Gesundheitszustandes zumindest teilweise auf der Grundlage des bestimmten Wertes der zweiten Messgröße für die aktuelle Schlafsitzung zu bestimmen;
Daten während einer oder mehrerer aufeinanderfolgender Schlafphasen zu generieren;
für jede der einen oder mehreren nachfolgenden Schlafsitzungen einen Wert der zweiten Messgröße zu bestimmen;
einen aktualisierten Schweregrad des Gesundheitszustands auf der Grundlage zumindest teilweise der bestimmten Werte der zweiten Messgröße für jede der einen oder mehreren nachfolgenden Schlafphasen zu bestimmen; und
auf der Grundlage zumindest teilweise des aktualisierten Schweregrads des Gesundheitszustandes zu veranlassen, dass die Maßnahme durchgeführt wird;
wobei optional:
die Maßnahme Übermitteln einer Empfehlung für die zukünftige Therapie an den Anwender (210) beinhaltet, zumindest teilweise auf der Grundlage des aktualisierten Schweregrads des Gesundheitszustandes; und/oder
als Reaktion auf den bestimmten Wert der zweiten Messgröße für jede der ein oder mehreren nachfolgenden Schlafsitzungen, der darauf hinweist, dass sich der Schweregrad des Gesundheitszustandes erhöht hat, die Maßnahme Anpassen einer oder mehrerer Einstellungen des Beatmungstherapiesystems (120) beinhaltet, um eine beabsichtigte Therapiewirkung des Beatmungstherapiesystems (120) zu erhöhen; und/oder
als Reaktion auf den bestimmten Wert der zweiten Messgröße für jede der einen oder mehreren nachfolgenden Schlafsitzungen, der darauf hinweist, dass sich der Schweregrad des Gesundheitszustandes erhöht hat, die Maßnahme Übermitteln einer Therapieempfehlung an den Anwender (210) oder an einen Dritten beinhaltet; und/oder
wobei als Reaktion auf den bestimmten Wert der zweiten Messgröße für jede der einen oder mehreren nachfolgenden Schlafsitzungen, der auf eine Verringerung des Schweregrades des Gesundheitszustandes hinweist, die Maßnahme Fortsetzen der Verwendung des Beatmungstherapiesystems (120) mit den aktuellen Einstellungen oder Anpassen einer oder mehrerer Einstellungen des Beatmungstherapiesystems (120) beinhaltet, um eine beabsichtigte Therapiewirkung des Beatmungstherapiesystems zu verringern.

9. System (100) nach einem der Ansprüche 1 bis 8, wobei der eine oder die mehreren Prozessoren (112) des Steuerungssystems (110) ferner so konfiguriert sind, dass sie die maschinenlesbaren Anweisungen ausführen, um:
zusätzliche, mit dem Anwender (210) in Zusammenhang stehende Daten im Wachzustand zu empfangen;
die zusätzlichen Daten zu analysieren, um einen Wert für eine dritte, mit dem Gesundheitszustand in Zusammenhang stehende Messgröße zu bestimmen; und
zu veranlassen, dass die Maßnahme zumindest teilweise auf der Grundlage des bestimmten Wertes der dritten, mit dem Gesundheitszustand in Zusammenhang stehenden Messgröße durchgeführt wird;
wobei optional die zusätzlichen Daten ein Alter des Anwenders (210), ein biologisches Geschlecht des Anwenders (210), ein soziales Geschlecht des Anwenders (210), einen geografischen Standort des Anwenders (210), eine Größe des Anwenders (210), ein Gewicht des Anwenders (210), einen Halsumfang des Anwenders (210), medizinische Informationen in Zusammenhang mit dem Anwender (210), einen Raucherstatus des Anwenders (210), einen Beruf des Anwenders (210), Antworten des Anwenders (210) auf einen Fragebogen, Art und Weise, wie der Anwender (210) die Antworten auf den Fragebogen gibt, oder eine beliebige Kombination davon beinhalten.

10. System (100) nach Anspruch 9, wobei der eine oder die mehreren Prozessoren (112) des Steuerungssystems (110) ferner so konfiguriert sind, dass sie die maschinenlesbaren Anweisungen ausführen, um:
den bestimmten Wert der zweiten Messgröße zumindest teilweise auf der Grundlage des bestimmten Wertes der dritten Messgröße zu modifizieren; und
zu veranlassen, dass die Maßnahme zumindest teilweise auf der Grundlage des geänderten Wertes der zweiten Messgröße durchgeführt wird;
wobei optional:
der Wert der zweiten Messgröße mit einer Vielzahl von Gesundheitszuständen in Zusammenhang steht und wobei der eine oder die mehreren Prozessoren (112) des Steuerungssystems (110) ferner so konfiguriert sind, dass sie die maschinenlesbaren Anweisungen ausführen, um eine Identität des Gesundheitszustands des Anwenders (210) aus der Vielzahl der Gesundheitszustände zumindest teilweise auf der Grundlage des bestimmten Wertes der dritten Messgröße zu bestimmen; und/oder
der Wert der dritten Messgröße mit einer Vielzahl von Gesundheitszuständen in Zusammenhang steht und wobei der eine oder die mehreren Prozessoren (112) des Steuerungssystems (110) ferner so konfiguriert sind, dass sie die maschinenlesbaren Anweisungen ausführen, um eine Identität des Gesundheitszustands des Anwenders (210) aus der Vielzahl der Gesundheitszustände zumindest teilweise auf der Grundlage des bestimmten Wertes der zweiten Messgröße zu bestimmen; und/oder
der eine oder die mehreren Prozessoren (112) des Steuerungssystems (110) ferner konfiguriert sind, die maschinenlesbaren Anweisungen auszuführen, um:
eine Identität des Gesundheitszustands des Anwenders (210) zumindest teilweise auf der Grundlage des bestimmten Wertes der dritten Messgröße zu bestimmen; und
einen Schweregrad des Gesundheitszustands des Anwenders (210) zumindest teilweise auf der Grundlage des bestimmten Wertes der zweiten Messgröße zu bestimmen; und/oder
der eine oder die mehreren Prozessoren (112) des Steuerungssystems (110) ferner konfiguriert sind, die maschinenlesbaren Anweisungen auszuführen, um:
eine Identität des Gesundheitszustands des Anwenders (210) zumindest teilweise auf der Grundlage des bestimmten Wertes der dritten Messgröße zu bestimmen; und
einen Schweregrad des Gesundheitszustands des Anwenders (210) zumindest teilweise auf der Grundlage des bestimmten Wertes der zweiten Messgröße zu bestimmen.

11. System (100) nach einem der Ansprüche 1 bis 10, wobei der eine oder die mehreren Prozessoren (112) des Steuerungssystems (110) ferner so konfiguriert sind, dass sie die maschinenlesbaren Anweisungen ausführen, um eine Identität des Gesundheitszustands, einen Schweregrad des Gesundheitszustands oder beides zumindest teilweise auf der Grundlage (i) des bestimmten Wertes der zweiten Messgröße und (ii) zusätzlicher Daten zu bestimmen;
wobei optional die zusätzlichen Daten demografische Informationen, die mit dem Anwender (210) in Zusammenhang stehen, medizinische Informationen, die mit dem Anwender (210) in Zusammenhang stehen, Audiodaten, die mit dem Anwender (210) in Zusammenhang stehen, oder eine beliebige Kombination davon beinhalten; und
wobei optional:
die demografischen Informationen ein Alter des Anwenders (210), ein biologisches Geschlecht des Anwenders (210), ein soziales Geschlecht des Anwenders (210), einen geografischen Standort des Anwenders (210), eine Größe des Anwenders (210), ein Gewicht des Anwenders (210), einen Halsumfang des Anwenders (210), einen Beruf des Anwenders (210) oder eine beliebige Kombination davon beinhalten; und/oder
die medizinischen Informationen des Anwenders (210) aus einer Krankengeschichte des Anwenders (210) erhalten werden; und/oder
die mit dem Anwender (210) in Zusammenhang stehenden Audiodaten Antworten des Anwenders (210) auf einen Fragebogen beinhalten.

12. System (100) nach einem der Ansprüche 1 bis 11, wobei die zweite Messgröße einen Stresspegel des Anwenders (210) anzeigt und eine Herzfrequenz, eine Herzfrequenzvariabilität, eine Hautleitfähigkeit, eine arterielle Pulsgeschwindigkeit, eine arterielle Pulsform, ein arterielles Pulsvolumen, eine arterielle Pulsamplitude oder eine Kombination davon beinhaltet; wobei optional die Maßnahme als Reaktion auf die zweite Messgröße, die einen erhöhten Stresspegel des Anwenders (210) während eines Apnoe-Ereignisses anzeigt, Erhöhen eines Drucks der Druckluft beinhaltet.

13. System (100) nach einem der Ansprüche 1 bis 12, wobei der bestimmte Wert der zweiten Messgröße auf ein erhöhtes Stressniveau des Anwenders (210) hinweist;
wobei optional:
die Maßnahme (i) als Reaktion auf das Auftreten einer Apnoe bei dem Anwender (210) Erhöhen eines Drucks der Druckluft, (ii) als Reaktion auf das Ende der Apnoe Verringern des Drucks der Druckluft oder (iii) sowohl (i) als auch (ii) beinhaltet; und/oder
während des Betriebs des Beatmungstherapiesystems das Beatmungstherapiesystem (120) so konfiguriert ist, dass ein Druck der Druckluft innerhalb einer ersten Zeitspanne von einem Anfangsdruck auf einen Arbeitsdruck erhöht wird;
wobei optional die Maßnahme Verlä ngern einer Anstiegszeit der dem Atemweg des Anwenders (210) zugeführten Druckluft beinhaltet, sodass der Druck der Druckluft vom Anfangsdruck auf den Arbeitsdruck in einem zweiten Zeitraum ansteigt, der größer ist als der erste Zeitraum; und
wobei optional die Maßnahme Ändern des Arbeitsdrucks auf einen geänderten Arbeitsdruck beinhaltet, der kleiner als der Arbeitsdruck ist.

14. System (100) nach einem der Ansprüche 1 bis 13, wobei der bestimmte Wert der zweiten Messgröße anzeigt, dass der Anwender (210) an einer Atemwegserkrankung leidet, und wobei die Maßnahme Erhöhen einer Luftfeuchtigkeit der dem Atemweg des Anwenders (210) zugeführten Druckluft beinhaltet; wobei optional die Atemwegserkrankung eine chronisch obstruktive Lungenerkrankung (COPD), Asthma, Allergien, eine Nasennebenhöhlenentzündung, Rhinitis oder eine Kombination davon beinhaltet.

## Revendications

1. Système (100) de surveillance d'une session de sommeil d'un utilisateur (210), le système (100) comprenant :
un système de thérapie respiratoire (120) incluant :
un dispositif de thérapie respiratoire (122) configuré pour fournir de l'air sous pression, le dispositif de thérapie respiratoire (122) incluant un boîtier présentant un ou plusieurs capteurs (130) positionnés dans celui-ci ; et
une interface utilisateur (124) couplée au dispositif de thérapie respiratoire (122) via un conduit (126), l'interface utilisateur (124) étant configurée pour engager l'utilisateur (210) et aider à diriger l'air sous pression fourni vers les voies respiratoires de l'utilisateur (210) ;
un dispositif de mémoire (114) stockant des instructions lisibles par machine ; et
un système de commande (110) couplé au dispositif de mémoire (114), le système de commande (110) incluant un ou plusieurs processeurs (112) configurés pour exécuter les instructions lisibles par machine pour :
déclencher une génération (502) de données associées à l'utilisateur (210), au cours d'une session de sommeil actuelle, les données incluant au moins une première partie générée par les un ou plusieurs capteurs (130) positionnés dans le boîtier du dispositif de thérapie respiratoire (122) ;
analyser (504) les données générées pour déterminer une valeur d'une première mesure associée à un état de trouble respiratoire du sommeil (TRS) ;
analyser (506) les données générées pour déterminer une valeur d'une deuxième mesure associée à un état de santé autre que l'état de TRS, l'état de santé incluant l'insomnie, dans lequel la deuxième mesure inclut un temps au lit total, un temps de sommeil total, un temps d'éveil total, une latence d'endormissement, un paramètre d'éveil après l'endormissement, une efficacité de sommeil, un indice de fragmentation, un laps de temps d'endormissement, une régularité de fréquence respiratoire, un temps d'endormissement, un temps d'éveil, un taux de perturbations de sommeil, un nombre de mouvements, ou toute combinaison de ceux-ci ;
**caractérisé en ce que** les un ou plusieurs processeurs sont en outre configurés pour exécuter les instructions lisibles par machine pour :
en réponse à la valeur déterminée de la deuxième mesure indiquant la présence d'insomnie, déclencher (508) l'exécution d'une action, l'action incluant l'ajustement d'un ou plusieurs réglages du système de thérapie respiratoire, l'ajustement incluant :
(i) la diminution de la pression de l'air sous pression en dessous d'une pression de thérapie souhaitée, et en réponse à la détermination que l'utilisateur s'est endormi, l'augmentation de la pression de l'air sous pression au moins jusqu'à la pression de thérapie souhaitée ; et/ou
(ii) l'augmentation du temps de montée de l'air sous pression, où le temps de montée est le temps nécessaire pour que la pression de l'air atteigne la pression de thérapie souhaitée à partir du début de l'utilisation du système de thérapie respiratoire.

2. Système (100) selon la revendication 1, dans lequel la valeur de la deuxième mesure est indicative d'une présence de l'affection, d'une gravité de l'affection, ou des deux ; et/ou
dans lequel les un ou plusieurs processeurs (112) du système de commande (110) sont en outre configurés pour exécuter les instructions lisibles par machine afin de déterminer une identité de l'affection sur la base au moins en partie de la valeur déterminée de la deuxième mesure, déterminer une gravité de l'affection sur la base au moins en partie de la valeur déterminée de la deuxième mesure, ou les deux ; et/ou
dans lequel la première mesure est associée uniquement au TRS, et la deuxième mesure est associée uniquement à l'affection ; et/ou
dans lequel la première mesure inclut un signal respiratoire, une fréquence respiratoire, un modèle respiratoire, une amplitude d'inspiration, une amplitude d'expiration, un rapport inspiration-expiration, la survenue d'un ou plusieurs événements, un nombre d'événements par heure, un modèle d'événements, une durée moyenne des événements, une plage de durées d'événements, un rapport entre le nombre d'événements différents, un stade de sommeil, un temps passé dans chacun d'une pluralité de stades de sommeil, un modèle de stades de sommeil, un indice d'apnéehypopnée (IAH), un score de sommeil, un score de thérapie, un temps de sommeil total, un temps au lit total, un temps de réveil, un temps de lever, un hypnogramme, un indice de perturbation de sommeil, un temps total de sommeil léger, un temps total de sommeil profond, un temps total de sommeil paradoxal, un temps total de sommeil sous thérapie, un temps total de sommeil sans thérapie, un nombre de réveils, une latence d'endormissement, ou toute combinaison de ceux-ci ; et/ou
dans lequel les un ou plusieurs capteurs (130) positionnés dans le boîtier du dispositif de thérapie respiratoire (122) incluent un capteur de débit (134), un capteur de pression (132), un microphone (140) ou toute combinaison de ceux-ci ; et/ou
dans lequel au moins une seconde partie des données est générée par un ou plusieurs capteurs (130) positionnés à l'extérieur du dispositif de thérapie respiratoire; et/ou
dans lequel la deuxième mesure est associée à la respiration de l'utilisateur (210) pendant la session de sommeil actuelle ; et/ou
dans lequel la deuxième mesure inclut une cadence de la respiration, une amplitude de la respiration de l'utilisateur (210), une constante de temps d'expiration, une forme d'expiration, une amplitude d'expiration, une vitesse d'expiration, une constante de temps d'inspiration, une forme d'inspiration, une amplitude d'inspiration, une vitesse d'inspiration, un volume courant, un taux de respiration, ou toute combinaison de ceux-ci.

3. Système (100) selon la revendication 2, dans lequel la deuxième mesure inclut la cadence de la respiration et l'affection est l'obésité, la bronchopneumopathie chronique obstructive (BPCO), la pneumonie, l'asthme, la respiration de Cheyne-Stokes, l'insuffisance cardiaque ou toute combinaison de celles-ci; facultativement, dans lequel la cadence de la respiration indique un essoufflement et est associée à la présence d'obésité ; et/ou
dans lequel la deuxième mesure inclut une forme d'expiration, et dans lequel l'affection est la présence d'une obstruction dans une voie respiratoire de l'utilisateur (210) ; et/ou
dans lequel la deuxième mesure inclut un volume courant, et dans lequel une instabilité du volume courant indique la présence d'une insuffisance cardiaque ou d'un risque de développer une insuffisance cardiaque ; facultativement, dans lequel le système de thérapie respiratoire (120) est initialement utilisé comme un système de pression positive des voies respiratoires, et dans lequel, en réponse au deuxième paramètre indiquant la présence d'une insuffisance cardiaque ou d'un risque de développer une insuffisance cardiaque, l'action inclut l'ajustement d'un ou plusieurs réglages du système de thérapie respiratoire (120) pour fonctionner comme un système de servo-ventilation adaptatif.

4. Système (100) selon l'une quelconque des revendications 1 à 3,
dans lequel, en réponse à la deuxième mesure indiquant la présence d'insomnie, l'action inclut la transmission d'une recommandation pour une thérapie future à l'utilisateur (210) ou à un tiers ;
facultativement dans lequel la thérapie future inclut une thérapie cognitivocomportementale ; et
l'affection inclut une affection cardiaque, et dans lequel la deuxième mesure inclut une variabilité de la fréquence cardiaque, un écart type des intervalles entre les battements cardiaques de l'utilisateur (210), une racine carrée moyenne des différences successives entre les battements cardiaques adjacents de l'utilisateur (210), un débit cardiaque, une structure des cavités atriales du cœur de l'utilisateur (210), un volume courant, une durée de l'apnée, une durée d'expiration, un gradient d'expiration, une enveloppe du signal de flux, une fréquence respiratoire, ou toute combinaison de ceux-ci ; et
facultativement, dans lequel le système de thérapie respiratoire (120) est initialement utilisé comme un système de pression positive des voies respiratoires, et en réponse à la deuxième mesure indiquant la présence d'une affection cardiaque, l'action inclut l'ajustement d'un ou plusieurs réglages du système de thérapie respiratoire (120) pour fonctionner comme un système de servo-ventilation adaptatif ; et/ou
l'affection cardiaque inclut une insuffisance cardiaque, un souffle au cœur, une augmentation du rythme cardiaque, une crise cardiaque, une fibrillation atriale, une arythmie cardiaque, une myocardite, une neuropathie autonome cardiaque, une inflammation chronique ou toute combinaison de ceux-ci.

5. Système (100) selon l'une quelconque des revendications 1 à 4, dans lequel :
l'action inclut la transmission d'une notification à l'utilisateur (210) ou à un tiers, l'affichage d'une notification sur un dispositif d'affichage électronique (172), la modification d'un ou plusieurs réglages du système de thérapie respiratoire, la transmission d'une recommandation de thérapie à l'utilisateur (210) ou au tiers, ou toute combinaison de ceux-ci ; et/ou
les données générées incluent des données indicatives de la pression de l'air sous pression, du flux de l'air sous pression, ou les deux ;
facultativement, dans lequel l'action inclut la modification du débit de l'air sous pression, l'administration d'un médicament dans les voies respiratoires de l'utilisateur (210) via l'air sous pression, ou les deux ; et
facultativement, dans lequel en réponse à la valeur déterminée de la deuxième mesure indiquant la présence d'insomnie, d'anxiété, de claustrophobie ou de toute combinaison de celles-ci, l'action inclut l'augmentation du temps de montée de l'air sous pression ; et/ou
dans lequel en réponse à la valeur déterminée de la deuxième mesure indiquant la présence de BPCO, d'asthme, d'allergies, d'une infection des sinus, d'une rhinite ou de toute combinaison de celles-ci, l'action inclut l'augmentation de l'humidité de l'air sous pression, l'administration du médicament dans les voies respiratoires de l'utilisateur (210), ou les deux.

6. Système (100) selon l'une quelconque des revendications 1 à 5, dans lequel les un ou plusieurs processeurs (112) du système de commande (110) sont en outre configurés pour exécuter les instructions lisibles par machine pour :
suivre la deuxième mesure sur une pluralité de sessions de sommeil ;
déterminer la valeur de la deuxième mesure pour chacune de la pluralité de sessions de sommeil ;
déterminer (i) une durée d'utilisation du système de thérapie respiratoire (120) pour chacune de la pluralité de sessions de sommeil, (ii) des valeurs d'un ou plusieurs réglages du système de thérapie respiratoire (120) pour chacune de la pluralité de sessions de sommeil, (iii) ou les deux ; et
identifier (i) une durée optimale d'utilisation du système de thérapie respiratoire, (ii) des valeurs optimales des un ou plusieurs réglages du système de thérapie respiratoire, ou (iii) les deux, pour traiter l'affection ; et/ou
dans lequel les un ou plusieurs processeurs (112) du système de commande (110) sont en outre configurés pour exécuter les instructions lisibles par machine pour :
recevoir des données historiques associées à une ou plusieurs sessions de sommeil précédentes de l'utilisateur (210) ;
analyser les données historiques afin de déterminer une valeur pour la deuxième mesure pour chacune des une ou plusieurs sessions de sommeil précédentes ; et
comparer la valeur déterminée de la deuxième mesure pour chacune des une ou plusieurs sessions de sommeil précédentes à la valeur déterminée de la deuxième mesure pour la séance de sommeil actuelle.

7. Système (100) selon la revendication 6, dans lequel :
l'affection inclut un niveau de stress de l'utilisateur (210), et dans lequel en réponse à la comparaison indiquant que le niveau de stress de l'utilisateur (210) a augmenté, l'action inclut une diminution d'une pression de l'air sous pression délivré à l'utilisateur (210) par le système de thérapie respiratoire ; et/ou
les un ou plusieurs processeurs (112) du système de commande (110) sont configurés pour exécuter les instructions lisibles par machine afin de provoquer l'exécution de l'action en réponse à la comparaison indiquant qu'une gravité de l'affection a augmenté ;
facultativement, dans lequel l'action inclut la transmission d'une notification à l'utilisateur (210) ou à un tiers, l'affichage d'une notification sur un dispositif d'affichage électronique (172), l'ajustement d'un ou plusieurs réglages du système de thérapie respiratoire, la transmission d'une recommandation de thérapie à l'utilisateur (210) ou au tiers, ou toute combinaison de ceux-ci ;
facultativement, dans lequel la recommandation de thérapie inclut une recommandation de consulter un médecin, une recommandation de consulter un dentiste, une recommandation d'utiliser un dispositif séparé pour effectuer une mesure indicative d'une présence ou d'une gravité de l'affection, une recommandation d'utiliser un concentrateur d'oxygène portable, ou toute combinaison de ceux-ci ; et facultativement
dans lequel le dispositif séparé est un oxymètre de pouls configuré pour mesurer une saturation en oxygène de l'utilisateur (210), un tensiomètre (180) configuré pour surveiller une pression artérielle de l'utilisateur (210), un moniteur de fréquence cardiaque configuré pour mesurer une fréquence cardiaque de l'utilisateur (210), un glucomètre configuré pour surveiller une glycémie de l'utilisateur (210), un dispositif d'électroencéphalographie configuré pour surveiller une activité cérébrale, un dispositif d'électrooculographie configuré pour surveiller des mouvements oculaires, un dispositif portable configuré pour mesurer des mouvements physiques de l'utilisateur (210), un capteur de matelas configuré pour mesurer des mouvements physiques de l'utilisateur (210), ou toute combinaison de ceux-ci.

8. Système (100) selon l'une quelconque des revendications 1 à 7, dans lequel les un ou plusieurs processeurs (112) du système de commande (110) sont en outre configurés pour exécuter les instructions lisibles par machine pour :
déterminer une gravité de l'affection sur la base au moins en partie de la valeur déterminée de la deuxième mesure pour la session de sommeil actuelle ;
générer des données au cours d'une ou plusieurs sessions de sommeil ultérieures ;
déterminer une valeur de la deuxième mesure pour chacune des une ou plusieurs sessions de sommeil ultérieures ;
déterminer une gravité mise à jour de l'affection sur la base au moins en partie des valeurs déterminées de la deuxième mesure pour chacune des une ou plusieurs sessions de sommeil ultérieures ; et
sur la base au moins en partie de la gravité mise à jour de l'affection, provoquer l'exécution de l'action ;
facultativement dans lequel :
l'action inclut la transmission d'une recommandation pour une thérapie future à l'utilisateur (210) sur la base au moins en partie de la gravité mise à jour de l'affection ; et/ou
en réponse à la valeur déterminée de la deuxième mesure pour chacune des une ou plusieurs sessions de sommeil ultérieures indiquant que la gravité de l'affection a augmenté, l'action inclut l'ajustement d'un ou plusieurs réglages du système de thérapie respiratoire (120) pour augmenter un effet thérapeutique prévu du système de thérapie respiratoire (120) ; et/ou
en réponse à la valeur déterminée de la deuxième mesure pour chacune des une ou plusieurs sessions de sommeil ultérieures indiquant que la gravité de l'affection a augmenté, l'action inclut la transmission d'une recommandation de thérapie à l'utilisateur (210) ou à un tiers ; et/ou
dans lequel en réponse à la valeur déterminée de la deuxième mesure pour chacune des une ou plusieurs sessions de sommeil ultérieures indiquant que la gravité de l'affection a diminué, l'action inclut la continuité d'utilisation du système de thérapie respiratoire (120) avec les réglages actuels, ou l'ajustement d'un ou plusieurs réglages du système de thérapie respiratoire (120) pour diminuer un effet thérapeutique prévu du système de thérapie respiratoire.

9. Système (100) selon l'une quelconque des revendications 1 à 8, dans lequel les un ou plusieurs processeurs (112) du système de commande (110) sont en outre configurés pour exécuter les instructions lisibles par machine pour :
recevoir des données supplémentaires associées à l'utilisateur (210) lorsqu'il est éveillé ;
analyser les données supplémentaires afin de déterminer la valeur d'une troisième mesure associée à l'affection ; et
provoquer l'exécution de l'action sur la base au moins en partie de la valeur déterminée de la troisième mesure associée à l'affection ;
facultativement, dans lequel les données supplémentaires incluent l'âge de l'utilisateur (210), le sexe de l'utilisateur (210), le genre de l'utilisateur (210), la localisation géographique de l'utilisateur (210), la taille de l'utilisateur (210), le poids de l'utilisateur (210), la taille du cou de l'utilisateur (210), des informations médicales associées à l'utilisateur (210), le tabagisme de l'utilisateur (210), la profession de l'utilisateur (210), les réponses de l'utilisateur (210) à un questionnaire, la manière dont l'utilisateur (210) fournit les réponses au questionnaire, ou toute combinaison de ceux-ci.

10. Système (100) selon la revendication 9, dans lequel les un ou plusieurs processeurs (112) du système de commande (110) sont en outre configurés pour exécuter les instructions lisibles par machine pour :
modifier la valeur déterminée de la deuxième mesure sur la base au moins en partie de la valeur déterminée de la troisième mesure ; et
provoquer l'exécution de l'action sur la base au moins en partie de la valeur modifiée de la deuxième mesure ;
facultativement dans lequel :
la valeur de la deuxième mesure est associée à une pluralité d'affections, et dans lequel les un ou plusieurs processeurs (112) du système de contrôle (110) sont en outre configurés pour exécuter les instructions lisibles par machine afin de déterminer une identité de l'affection de l'utilisateur (210) parmi la pluralité d'affections sur la base au moins en partie de la valeur déterminée de la troisième mesure ; et/ou
la valeur de la troisième mesure est associée à une pluralité d'affections, et dans lequel les un ou plusieurs processeurs (112) du système de contrôle (110) sont en outre configurés pour exécuter les instructions lisibles par machine afin de déterminer une identité de l'affection de l'utilisateur (210) parmi la pluralité d'affections sur la base au moins en partie de la valeur déterminée de la deuxième mesure ; et/ou
les un ou plusieurs processeurs (112) du système de commande (110) sont en outre configurés pour exécuter les instructions lisibles par machine pour :
déterminer une identité de l'affection de l'utilisateur (210) sur la base au moins en partie de la valeur déterminée de la troisième mesure ; et
déterminer une gravité de l'affection de l'utilisateur (210) sur la base au moins en partie de la valeur déterminée de la deuxième mesure ; et/ou
les un ou plusieurs processeurs (112) du système de commande (110) sont en outre configurés pour exécuter les instructions lisibles par machine pour :
déterminer une identité de l'affection de l'utilisateur (210) sur la base au moins en partie de la valeur déterminée de la troisième mesure ; et
déterminer une gravité de l'affection de l'utilisateur (210) sur la base au moins en partie de la valeur déterminée de la deuxième mesure .

11. Système (100) selon l'une quelconque des revendications 1 à 10, dans lequel les un ou plusieurs processeurs (112) du système de commande (110) sont en outre configurés pour exécuter les instructions lisibles par machine afin de déterminer une identité de l'affection, une gravité de l'affection, ou les deux, sur la base au moins en partie (i) de la valeur déterminée de la deuxième mesure et (ii) de données supplémentaires ;
facultativement, dans lequel les données supplémentaires incluent des informations démographiques associées à l'utilisateur (210), des informations médicales associées à l'utilisateur (210), des données audio associées à l'utilisateur (210), ou toute combinaison de celles-ci ; et
facultativement dans lequel :
les informations démographiques incluent l'âge de l'utilisateur (210), le sexe de l'utilisateur (210), le genre de l'utilisateur (210), la localisation géographique de l'utilisateur (210), la taille de l'utilisateur (210), le poids de l'utilisateur (210), la taille du cou de l'utilisateur (210), la profession de l'utilisateur (210), ou toute combinaison de ceux-ci ; et/ou
les informations médicales de l'utilisateur (210) sont obtenues à partir d'un historique médical de l'utilisateur (210) ; et/ou
les données audio associées à l'utilisateur (210) incluent les réponses de l'utilisateur (210) à un questionnaire.

12. Système (100) selon l'une quelconque des revendications 1 à 11, dans lequel la deuxième mesure est indicative du niveau de stress de l'utilisateur (210) et inclut une fréquence cardiaque, une variabilité de la fréquence cardiaque, une conductance cutanée, une vitesse du pouls artériel, une forme du pouls artériel, un volume du pouls artériel, une amplitude du pouls artériel ou toute combinaison de ceux-ci ; facultativement, dans lequel en réponse à la deuxième mesure indiquant un niveau de stress élevé de l'utilisateur (210) au cours d'un événement d'apnée, l'action inclut l'augmentation de la pression de l'air sous pression.

13. Système (100) selon l'une quelconque des revendications 1 à 12, dans lequel la valeur déterminée de la deuxième mesure indique un niveau de stress élevé de l'utilisateur (210) ;
facultativement dans lequel :
l'action inclut, (i) en réponse à un événement d'apnée de l'utilisateur (210), l'augmentation de la pression de l'air sous pression, (ii) en réponse à la fin de l'événement d'apnée, la diminution de la pression de l'air sous pression, ou (iii) les deux (i) et (ii) ; et/ou
pendant le fonctionnement du système de thérapie respiratoire, le système de thérapie respiratoire (120) est configuré pour augmenter la pression de l'air sous pression d'une pression initiale à une pression de travail sur une première période de temps ;
facultativement, dans lequel l'action inclut l'augmentation du temps de montée de l'air sous pression fourni aux voies respiratoires de l'utilisateur (210), de telle sorte que la pression de l'air sous pression augmente de la pression initiale à la pression de travail sur une seconde période de temps supérieure à la première ; et
facultativement, dans lequel l'action inclut la modification de la pression de travail pour être une pression de travail modifiée inférieure à la pression de travail.

14. Système (100) selon l'une quelconque des revendications 1 à 13, dans lequel la valeur déterminée de la deuxième mesure indique que l'utilisateur (210) souffre d'une affection respiratoire, et dans lequel l'action inclut l'augmentation de l'humidité de l'air sous pression fourni aux voies respiratoires de l'utilisateur (210) ; facultativement dans lequel l'affection respiratoire inclut une bronchopneumopathie chronique obstructive (BPCO), de l'asthme, des allergies, une infection des sinus, une rhinite ou toute combinaison de celles-ci.
